# EUROPÄISCHE PATENTSCHRIFT

(11) **EP 0 934 311 B1**
(45) Veröffentlichungstag und Bekanntmachung des Hinweises auf die Patenterteilung: **13.05.2009**
(21) Anmeldenummer: 97912102.7
(22) Anmeldetag: 02.10.1997
(51) Int. Cl.: C07D 405/04

(54) **NEUE HETEROCYCLYLMETHYL-SUBSTITUIERTE PYRAZOLDERIVATE UND IHRE VERWENDUNG IN DER BEHANDLUNG VON HERZ-KREISLAUF-ERKRANKUNGEN**
NEW HETEROCYCLYLMETHYL-SUBSTITUTED PYRAZOL DERIVATES
NOUVEAUX DERIVES DE PYRAZOL HETEROCYCLYLMETHYLE-SUBSTITUES

(30) Priorität: 14.10.1996 DE 19642319; 14.10.1996 DE 19642320; 14.10.1996 DE 19642322; 14.10.1996 DE 19642323
(43) Veröffentlichungstag der Anmeldung: 11.08.1999
(60) Teilanmeldung: 05022495.5
(73) Patentinhaber: Bayer HealthCare AG, 51368 Leverkusen (DE)
(72) Erfinder: STRAUB, Alexander, D-42113 Wuppertal (DE); FÜRSTNER, Chantal, D-45470 Mülheim (DE); NIEWÖHNER, Ulrich, D-42929 Wermelskirchen (DE); JAETSCH, Thomas, D-50668 Köln (DE); FEURER, Achim, D-51519 Odenthal (DE); KAST, Raimund, D-42389 Wuppertal (DE); STASCH, Johannes-Peter, D-42651 Solingen (DE); PERZBORN, Elisabeth, D-42327 Wuppertal (DE); HÜTTER, Joachim, D-42349 Wuppertal (DE); DEMBOWSKY, Klaus, D-42115 Wuppertal (DE); ARLT, Dieter, D-32657 Lemgo (DE)
(86) Internationale Anmeldenummer: PCT/EP1997/005432
(87) Internationale Veröffentlichungsnummer: WO 1998/016507

(56) Entgegenhaltungen:
- EP-A- 0 135 781
- EP-A- 0 220 573
- EP-A- 0 667 345
- WO-A-96/20192
- DE-A- 2 503 815
- CHEMICAL ABSTRACTS, vol. 125, no. 3, 15.Juli 1996 Columbus, Ohio, US; abstract no. 33633m, S. GUO ET AL.: "Preparation of condensed 1-benzyl-3-arylpyrazole derivatives as blood platelet aggergation inhibitors." Seite 903; Spalte 1; XP002056085 & CN 1 112 926 A (YONGXIN PHARMACEUTICAL INDUSTRY CO., LTD., PEOP. REP. CHINA) 6.Dezember 1995
- S.-M. YU ET AL.: "Inhibition of Platelet Function by A02131-1, a Novel Inhibitor of cGMP-Specific Phosphodiesterase, In Vitro and In Vivo" BLOOD, Bd. 87, Nr. 9, 1.Mai 1996, Seiten 3758-3767, XP002056082
- C.-C. WU ET AL.: "YC-1 inhibited human platelet aggregation through NO-independent activation of soluble guanylate cyclase" BRITISH JOURNAL OF PHARMACOLOGY , Bd. 116, Nr. 3, 1995, Seiten 1973-1978, XP002056083
- C.R. SELF ET AL.: "Romazarit: A Potential Disease-Modifying Antirheumatic Drug" JOURNAL OF MEDICINAL CHEMISTRY., Bd. 34, Nr. 2, 1991, WASHINGTON US, Seiten 772-777, XP002056084
- G. CAPOZZI ET AL.: "Neighbouring Group Participation in Electrophilic Addition to Acetylenes. 5_methylene Oxazolines from 3-Benzamidopropyne." TETRAHEDRON LETTERS., Bd. 22, Nr. 34, 1981, OXFORD GB, Seiten 3325-3328, XP002064296

## Beschreibung

Die vorliegende Erfindung betrifft neue Heterocyclylmethyl-substituierte Pyrazol-Derivate, Verfahren zu ihrer Herstellung und ihre Verwendung als Arzneimittel, insbesondere als Arzneimittel zur Behandlung von Herz-Kreislauf-Erkrankungen.

Es ist bereits bekannt, daß 1-Benzyl-3-(substituierte heteroaryl)-kondensierte Pyrazol-Derivate die stimulierte Thrombozytenaggregation in vitro inhibieren (vgl. EP 667 345 A1).

Die vorliegende Erfindung betrifft in der mit III (römisch drei) bezeichneten Ausführungsform neue 3-Heterocyclyl-substituierte Pyrazolderivate der allgemeinen Formel (III-I) 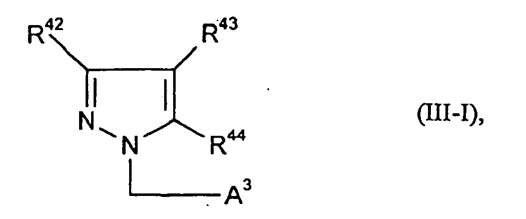 in welcher
- R⁴²: für Imidazolyl, Oxazolyl, Thiazolyl oder Oxadiazolyl steht, die gegebenenfalls bis zu 2-fach gleich oder verschieden durch Ethoxycarbonyl, Phenyl oder durch Methyl oder Ethyl substituiert sind, wobei die Alkylreste ihrerseits durch Hydroxy, Chlor, Ethoxycarbonyl, Oxycarbonylmethyl oder Methoxy substituiert sein können,
- R⁴³ und R⁴⁴: gemeinsam unter Einbezug der Doppelbindung für Phenyl stehen, das gegebenenfalls durch Nitro substituiert ist,
- A³: für Phenyl oder Fluor-substituiertes Phenyl oder Pyrimidyl steht,
und deren Isomere und Salze.

Die Erfindung betrifft weiterhin Verfahren zur Herstellung der erfindungsgemäßen Verbindungen der allgemeinen Formel (III-I), **dadurch gekennzeichnet, daß** man

### [A3] Verbindungen der allgemeinen Formel (III-II)

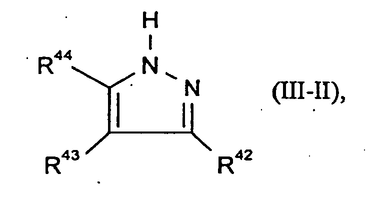

in welcher
R⁴², R⁴³ und R⁴⁴ die oben angegebene Bedeutung haben,
mit Verbindungen der allgemeinen Formel (III-III)

D³-CH₂-A³ (III-III),

in welcher
- A³: die oben angegebene Bedeutung hat,
und
- D³: für Triflat oder Halogen, vorzugsweise für Brom steht,
in inerten Lösemitteln, gegebenenfalls in Anwesenheit einer Base umsetzt,
oder

### [B3] Verbindungen der allgemeinen Formel (III-IV)

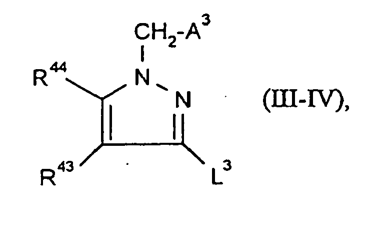

in welcher
A³, R⁴³ und R⁴⁴ die oben angegebene Bedeutung haben,
und
- L³: für einen Rest der Formel -SnR⁵⁵R⁵⁶R⁵⁷, ZnR⁵⁸, Iod, Brom oder Triflat steht,
worin
R⁵⁵, R⁵⁶ und R⁵⁷ gleich oder verschieden sind und geradkettiges oder verzweigtes Alkyl mit bis zu 4 Kohlenstoffatomen bedeuten,
und
R⁵⁸ Halogen bedeutet,
mit Verbindungen der allgemeinen Formel (III-V)

R⁴²-T³ (III-V),

in welcher
- R⁴²: die oben angegebene Bedeutung hat
und
im Fall L³ = SnR⁵⁵R⁵⁶R⁵⁷ oder ZnR⁵⁸
- T³: für Triflat oder für Halogen, vorzugsweise für Brom steht,
und
im Fall L³ = Jod, Brom oder Triflat
- T³: für einen Rest der Formel SnR^{55'}R^{56'}R^{57'}, ZnR^{58'} oder BR⁵⁹R⁶⁰ steht,
worin
R^{55'}, R^{56'}, R^{57'} und R^{58'} die oben angebene Bedeutung von R⁵⁵, R⁵⁶, R⁵⁷ und R⁵⁸ haben und mit dieser gleich oder verschieden sind,
R⁵⁹ und R⁶⁰ gleich oder verschieden sind und Hydroxy, Aryloxy mit 6 bis 10 Kohlenstoffatomen oder geradkettiges oder verzweigtes Alkyl oder Alkoxy mit jeweils bis zu 5 Kohlenstoffatomen bedeuten, oder gemeinsam einen 5- oder 6-gliedrigen carbocyclischen Ring bilden,
in einer palladiumkatalysierten Reaktion in inerten Lösemitteln umsetzt,
oder
[C3] im Fall R⁴² = 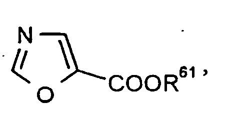 in welchen
- R⁶¹: für geradkettiges oder verzweigtes Alkyl mit bis zu 4 Kohlenstoffatomen steht,

### Verbindungen der allgemeinen Formel (III-VI)

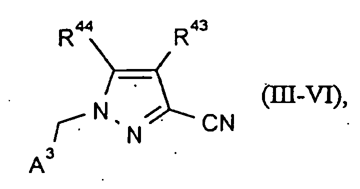

in welcher
A³, R⁴³ und R⁴⁴ die oben angegebene Bedeutung haben,
mit Diazoverbindungen der allgemeinen Formel (III-VII) 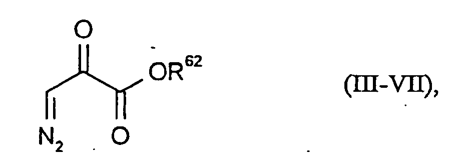 in welcher
- R⁶²: für geradkettiges oder verzweigtes Alkyl mit bis zu 4 Kohlenstoffatomen steht,
in Gegenwart von Kupfersalzen oder Rhodiumsalzen zu Verbindungen der allgemeinen Formel (III-Ia) 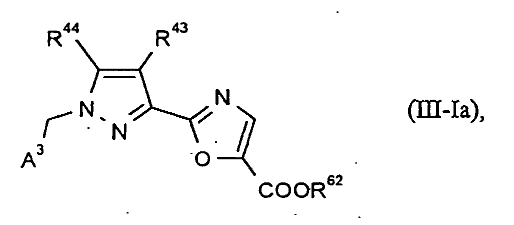 in welcher
A³, R⁴³, R⁴⁴ und R⁶² die oben angegebene Bedeutung haben,
umsetzt,
[D3] im Fall R⁴² = 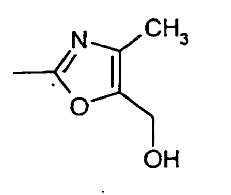 Verbindungen der allgemeinen Formel (III-VIII) 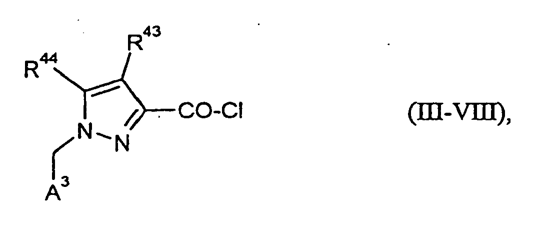 in welcher
A³, R⁴³ und R⁴⁴ die oben angegebene Bedeutung haben,
entweder direkt durch Umsetzung mit der Verbindung der Formel (III-IX) 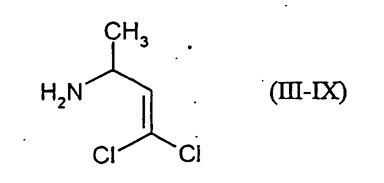 in dem System NaOCO-CH₃/N-Methylpyrrolidin
in die Verbindungen der allgemeinen Formel (III-Ib) 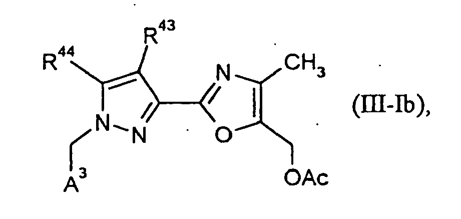 in welcher
R⁴³, R⁴⁴ und A³ die oben angegebene Bedeutung haben,
überführt,
und anschließend durch Einwirkung von Kaliumhydroxid in Methanol die Acetylgruppe abspaltet,
oder
zunächst durch Umsetzung der Verbindungen der allgemeinen Formel (III-VIII) mit der Verbindung der Formel (III-IX) die Verbindungen der allgemeinen Formel (III-X) 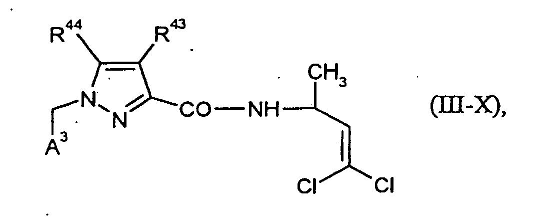 in welcher
R⁴³, R⁴⁴ und A³ die oben angegebene Bedeutung haben,
herstellt,
und in einem weiteren Schritt durch Einwirkung von Kaliumhydroxid die Hydroxymethylverbindungen herstellt,
und gegebenenfalls durch eine Alkylierung nach üblichen Methoden in die entsprechende Alkoxyverbindungen überführt,
oder

### [E3] Verbindungen der allgemeinen Formel (III-XI)

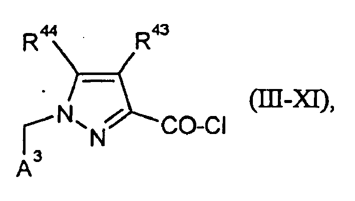

in welcher
A³, R⁴³ und R⁴⁴ die oben angegebene Bedeutung haben,
durch Umsetzung mit der Verbindung der Formel (III-XII) 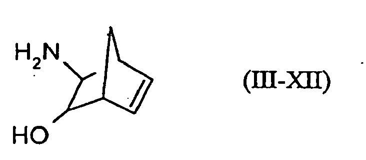 die Verbindungen der allgemeinen Formel (III-XIII) 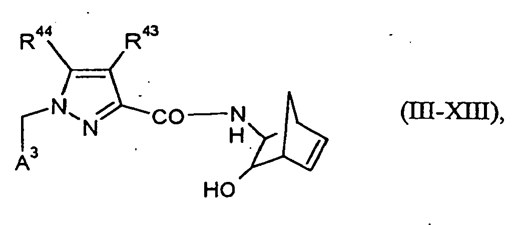 in welcher
A³, R⁴³ und R⁴⁴ die oben angegebene Bedeutung haben,
herstellt,
und anschließend im Sinne einer Retro-Diels-Alderreaktion umsetzt (vgl. J. Org. Chem. 1988, 58, 3387-90)
oder

### [F3] Verbindungen der allgemeinen Formel (III-XIV)

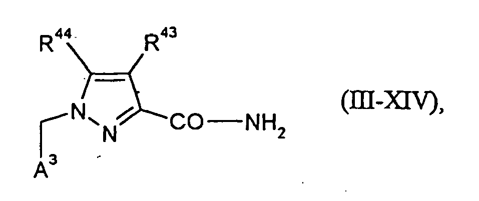

in welcher
A³, R⁴³ und R⁴⁴ die oben angegebene Bedeutung haben,
mit Verbindungen der allgemeinen Formel (III-XV)

Br-CH₂-CO-R⁶³ (III-XV),

in welcher
- R⁶³: geradkettiges oder verzweigtes Alkyl oder Alkoxycarbonyl mit jeweils bis zu 4 Kohlenstoffatomen bedeutet,
in inerten Lösemitteln zu den Verbindungen der allgemeinen Formel (III-Ic) 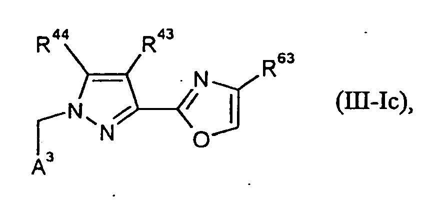 in welcher
A³, R⁴³, R⁴⁴ und R⁶³ die oben angegebene Bedeutung haben,
umsetzt,
(vgl. Oxazoles, J. Wiley/New York, 1986, S. 11/12),
und im Fall der Ester (R⁶³ = CO₂-(C₁-C₄-Alkyl), eine Reduktion nach üblichen Methoden zu den entsprechenden Hydroxymethylverbindungen durchführt,
oder

### [G3] im Fall R⁴² =

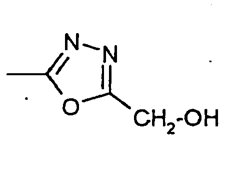

Carbonsäuren der allgemeinen Formel (III-XVI) 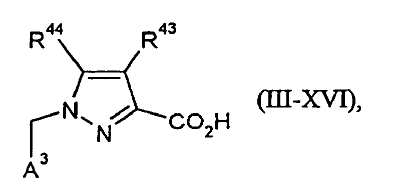 in welcher
A³, R⁴³ und R⁴⁴ die oben angegebene Bedeutung haben,
mit Hydrazinhydrat zunächst in die Verbindungen der allgemeinen Formel (III-XVII) 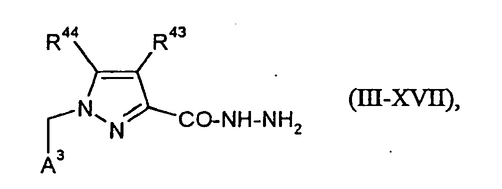 in welcher
A³, R⁴³ und R⁴⁴ die oben angegebene Bedeutung haben,
überführt,
in einem weiteren Schritt mit der Verbindung der Formel (III-XVIII)

Cl-CO-CH₂-Cl (III-XVIII)

die Verbindungen der allgemeinen Formel (III-XIX) 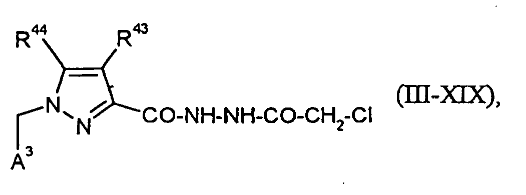 in welcher
A³, R⁴³ und R⁴⁴ die oben angegebene Bedeutung haben,
herstellt,
anschließend unter Einwirkung von Phosphoroxytrichlorid eine Cyclisierung zu den Verbindungen der allgemeinen Formel (III-Id) 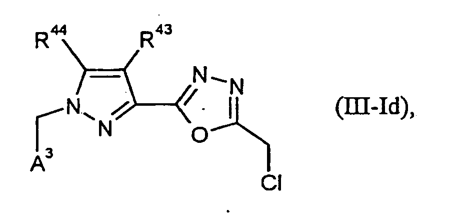 in welcher
A³, R⁴³ und R⁴⁴ die oben angegebene Bedeutung haben,
durchführt,
und wie oben bereits beschrieben über die Stufe der entsprechenden -CH₂-O-CO-CH₃ substituierten Verbindungen die -CH₂-OH substituierten Verbindungen herstellt, (vgl. Arzn. Forsch. 45 (1995) 10, 1074-1078),
oder
[H3] im Fall, daß R⁴² für einen Rest der Formel 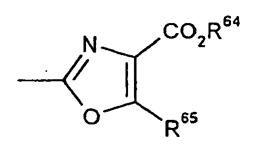 steht,
worin
- R⁶⁴: Wasserstoff oder geradkettiges oder verzweigtes Alkyl mit bis zu 4 Kohlenstoffatomen bedeutet und
- R⁶⁵: den Bedeutungsumfang der oben unter dem heterocyclischen Rest R⁴² aufgeführten Untersubstituenten umfaßt
Verbindungen der allgemeinen Formel (III-XX) 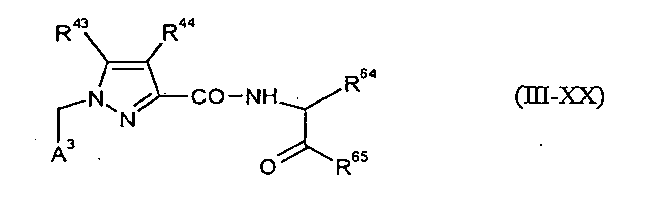 in welcher
A³, R⁴³, R⁴⁴, R⁶⁴ und R⁶⁵ die oben angegebene Bedeutung haben
im System PPh₃/J₂ in Anwesenheit einer Base, vorzugsweise mit Triethylamin umsetzt
oder

### [13] im Fall, daß R⁴⁵ für die Gruppe der Formel

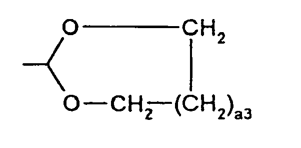

steht, worin a3 die oben angegebene Bedeutung hat
Verbindungen der allgemeinen Formel (III-XXI) 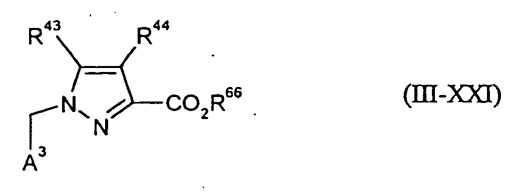 in welcher
A³, R⁴³ und R⁴⁴ die oben angegebene Bedeutung haben und
- R⁶⁶: die oben angegebene Bedeutung von R⁶⁴ hat und mit dieser gleich oder verschieden ist,
entweder zunächst durch Reduktion nach üblichen Methoden in die Verbindungen der allgemeinen Formel (III-XXII) 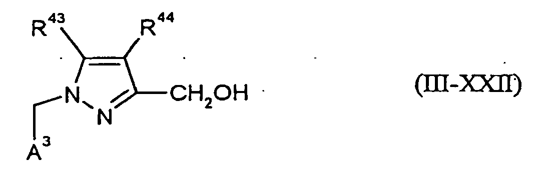 in welcher
A³, R⁴³ und R⁴⁴ die oben angegebene Bedeutung haben,
überführt und anschließend durch Oxidation die Verbindungen der allgemeinen Formel (III-XXIII) 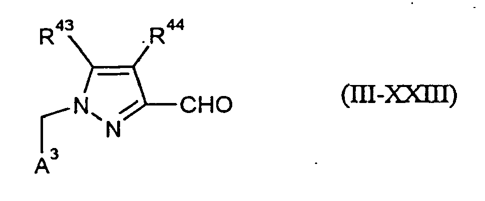 in welcher
A³, R⁴³ und R⁴⁴ die oben angegebene Bedeutung haben,
herstellt oder
direkt die Verbindungen der allgemeinen Formel (III-XXI) durch Reduktion in die Verbindungen der allgemeinen Formel (III-XXIII) überführt
und abschließend mit 1,2- oder 1,3-Dihydroxyverbindungen nach klassischen Methoden umsetzt
oder

### [J3] im Fall, daß R⁴² für den Rest der Formel

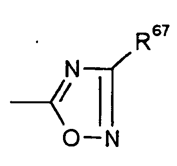

steht,
worin
- R⁶⁷: die oben angegebene Bedeutung von R⁶⁵ und mit dieser gleich oder verschieden ist,
entweder Verbindungen der allgemeinen Formel (III-XXIV) 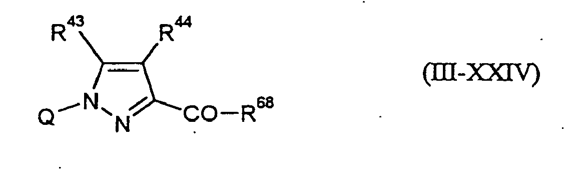 in welcher
R⁴³ und R⁴⁴ die oben angegebene Bedeutung haben und
- Q: für Wasserstoff oder für den -CH₂-A³-Rest steht und
- R⁶⁸: für Halogen oder geradkettiges oder verzweigtes Alkoxy mit bis zu 4 Kohlenstoffatomen, vorzugsweise für Chlor, Methoxy oder Ethoxy steht
mit Verbindungen der allgemeinen Formel (III-XXV) 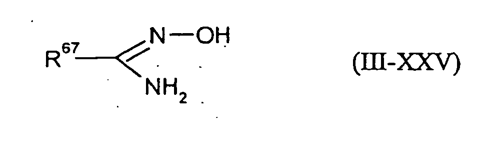 in welcher
- R⁶⁷: die oben angegebene Bedeutung hat
gegebenenfalls in Anwesenheit einer Base umsetzt und im Fall Q = H anschließend mit Verbindungen der allgemeinen Formel A³-CH₂-Br (III-XXVI), in welcher A die oben angegebene Bedeutung hat umsetzt oder
Verbindungen der allgemeinen Formel (III-XXVII) 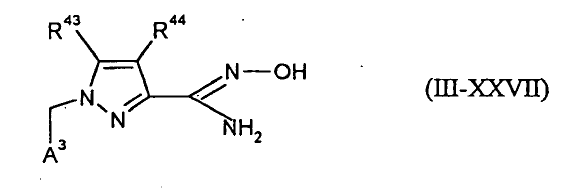 in welcher
A³, R⁴³ und R⁴⁴ die oben angegebene Bedeutung haben
mit Verbindungen der allgemeinen Formel (III-XXVIII)

R^{67'}-CO-R^{68'} (III-XXVIII)

in welcher
R^{67'} die oben angegebene Bedeutung von R⁶⁷ hat und mit dieser gleich oder verschieden ist
und
R^{68'} die oben angegebene Bedeutung von R⁶⁸ hat und mit dieser gleich oder verschieden ist gegebenenfalls in Anwesenheit einer Base umsetzt.
und im Fall der Reste -S(O)_{c3}NR⁵⁰R⁵¹ und -S(O)_{c3'}NR^{50'}R^{51'} ausgehend von den unsubstituierten Verbindungen der allgemeinen Formel (III-I) zunächst eine Umsetzung mit Thionylchlorid und abschließend mit der Aminkomponente durchführt.
und gegebenenfalls die unter R⁴², R⁴³, R⁴⁴ und/oder A³ aufgeführten Substituenten nach üblichen Methoden, vorzugsweise durch Reduktion, Oxidation, Abspaltung von Schutzgruppen und/oder nucleophiler Substitution variiert oder einführt.

Die oben beschriebenen erfindungsgemäßen Verfahren können durch folgendes Formelschema beispielhaft erläutert werden:

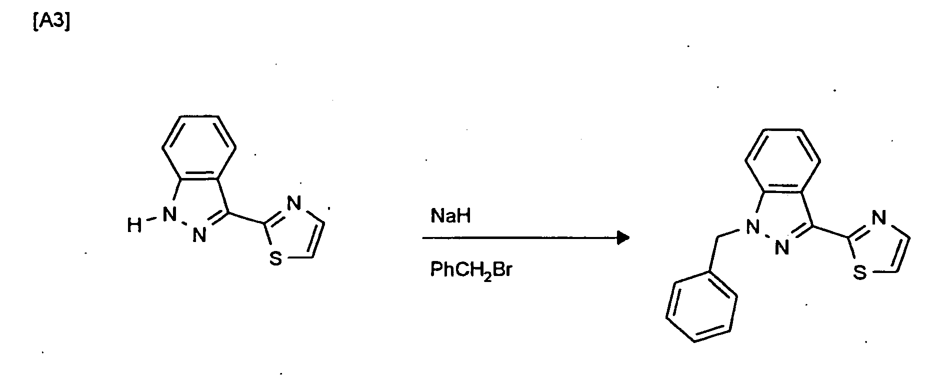

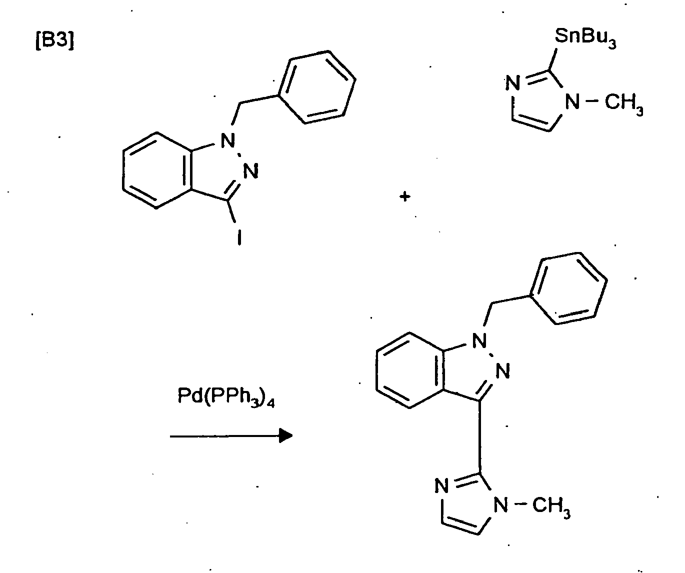

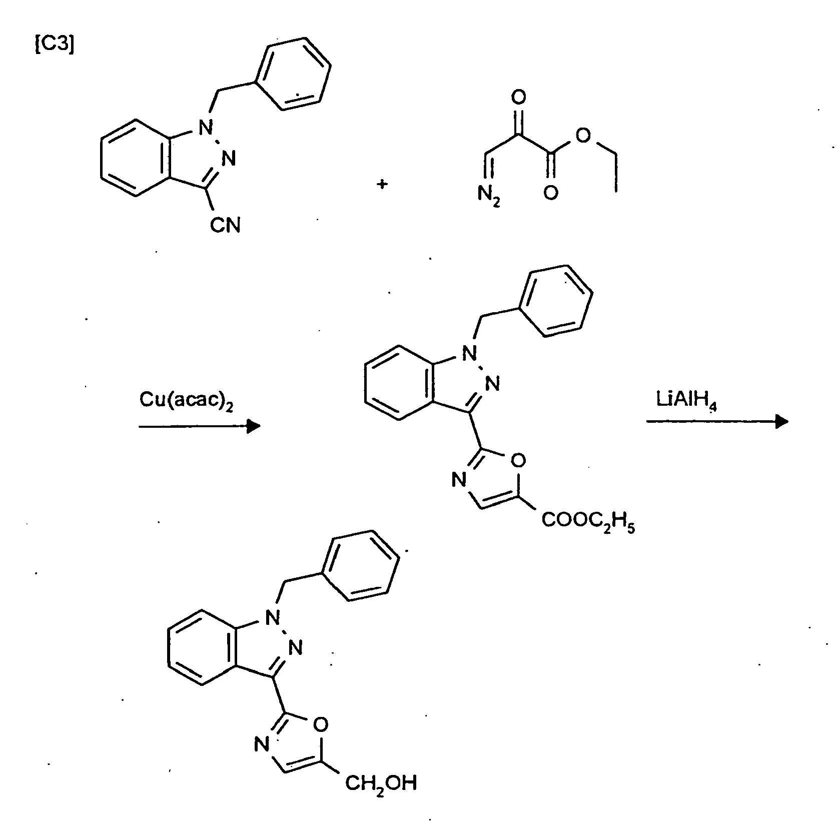

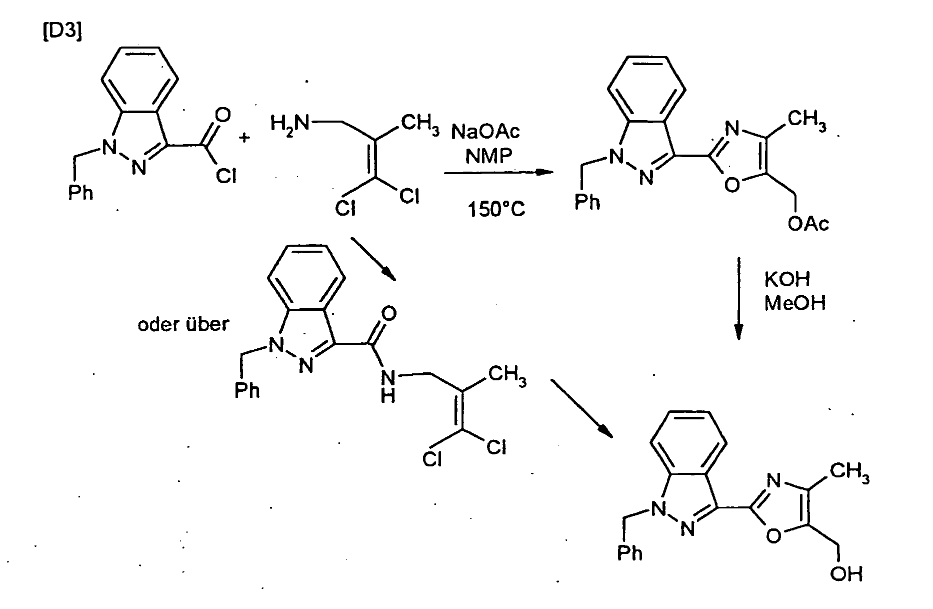

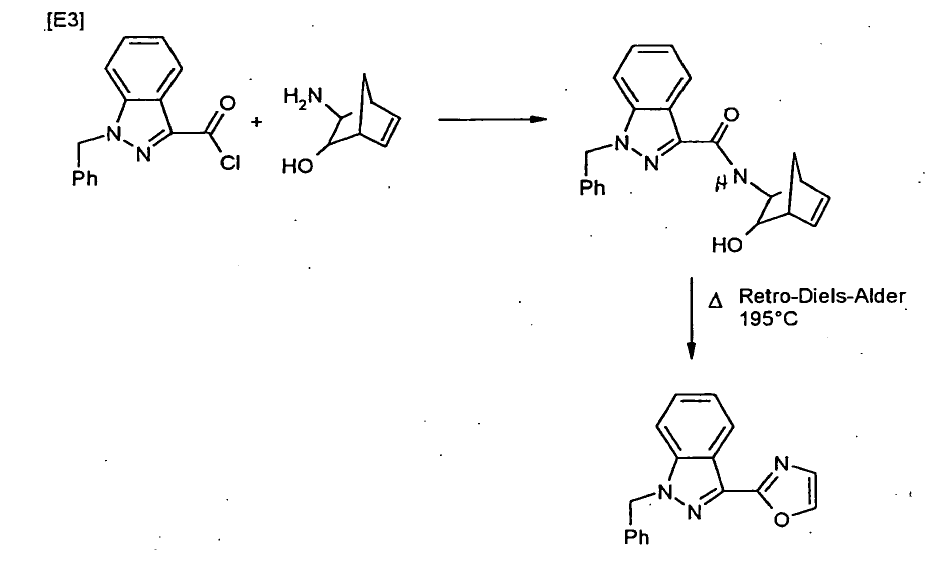

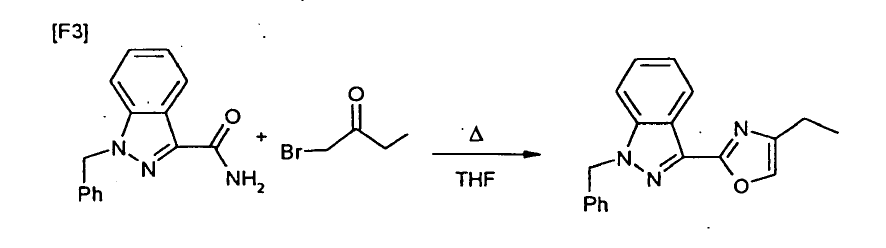

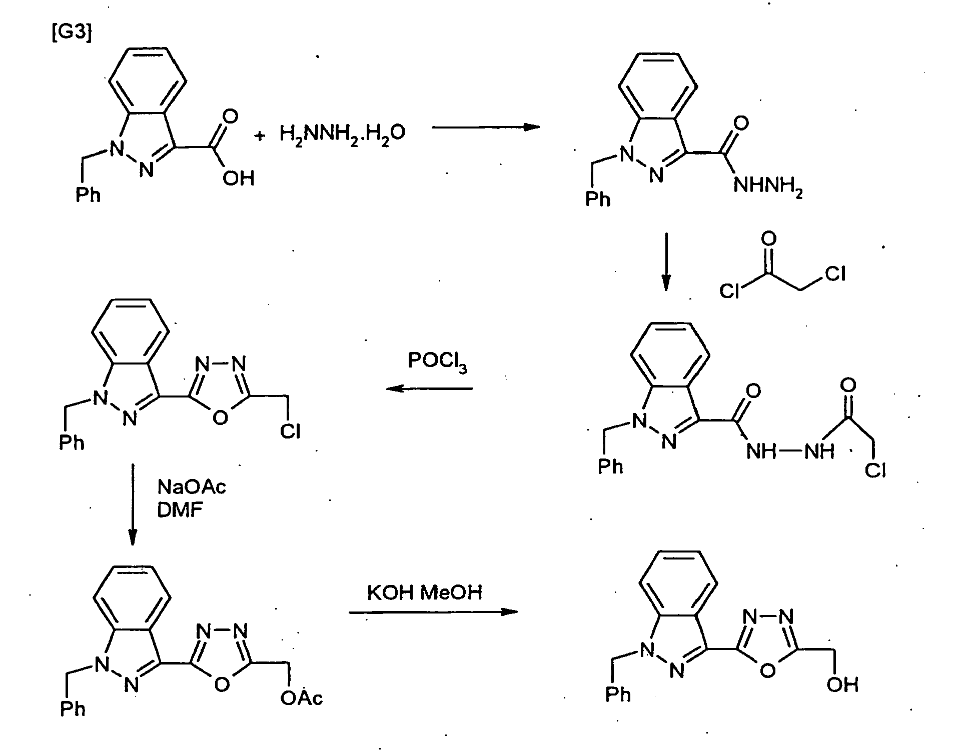

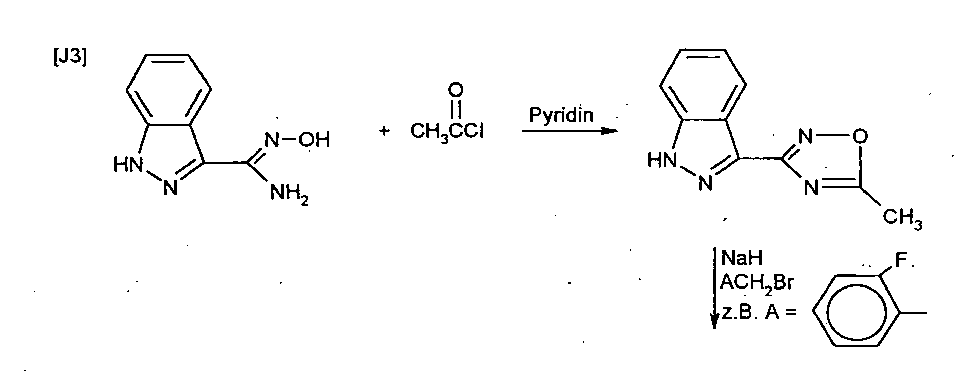

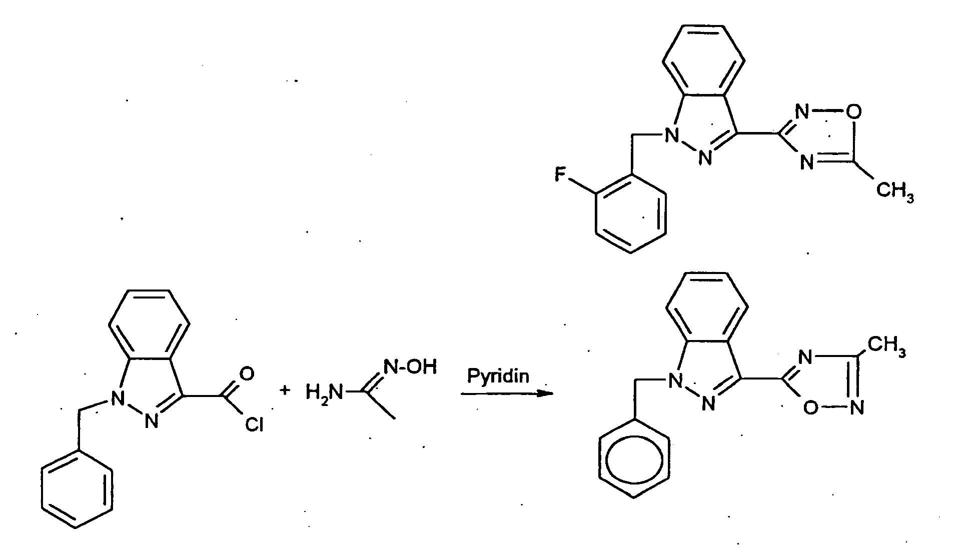

Als Lösemittel für die einzelnen Schritte des Verfahrens [A3] eignen sich hierbei inerte organische Lösemittel, die sich unter den Reaktionsbedingungen nicht verändern. Hierzu gehören Ether, wie Diethylether oder Tetrahydrofuran, Kohlenwasserstoffe wie Benzol, Xylol, Toluol, Hexan, Cyclohexan, oder Erdölfraktionen, Nitromethan, Dimethylformamid, Aceton, Acetonitril oder Hexamethylphosphorsäuretriamid. Ebenso ist es möglich, Gemische der Lösemittel einzusetzen. Besonders bevorzugt sind Tetrahydrofuran, Toluol oder Dimethylformamid.

Als Basen für das erfindungsgemäße Verfahren können im allgemeinen anorganische oder organische Basen eingesetzt werden. Hierzu gehören vorzugsweise Alkalihydroxide wie zum Beispiel Natriumhydroxid oder Kaliumhydroxid, Erdalkalihydroxide wie zum Beispiel Bariumhydroxid, Alkalicarbonate wie Natriumcarbonat oder Kaliumcarbonat, Erdalkalicarbonate wie Calciumcarbonat, oder Alkali- oder Erdalkalialkoholate wie Natrium- oder Kaliummethanolat, Natrium- oder Kaliumethanolat oder Kalium-tert.butylat, oder organische Amine (Trialkyl-(C₁-C₆)amine) wie Triethylamin, oder Heterocyclen wie 1,4-Diazabicyclo[2.2.2]oc-tan (DABCO), 1,8-Diazabicyclo[5.4.0]undec-7-en (DBU), Pyridin, Diaminopyridin, N-Methylpyrrolidon, Methylpiperidin oder Morpholin. Es ist auch möglich als Basen Alkalimetalle wie Natrium und deren Hydride wie Natriumhydrid einzusetzen. Bevorzugt sind Natrium- und Kaliumcarbonat, Triethylamin, Natriumhydrid und N-Methylpyrrilidon.

Die Base wird in einer Menge von 1 mol bis 5 mol, bevorzugt von 1 mol bis 3 mol, bezogen auf 1 mol der Verbindung der allgemeinen Formel (III-II) eingesetzt.

Die Umsetzung wird im allgemeinen in einem Temperaturbereich von 0°C bis 150°C, bevorzugt von +20°C bis +110°C durchgeführt.

Die Umsetzung kann bei normalen, erhöhtem oder bei erniedrigtem Druck durchgeführt werden (z.B. 0,5 bis 5 bar). Im allgemeinen arbeitet man bei Normaldruck.

Als Lösemittel für das Verfahren [B3] eignen sich hierbei inerte organische Lösemittel, die sich unter den Reaktionsbedingungen nicht verändern. Hierzu gehören Ether, wie Diethylether oder Tetrahydrofuran, DME, Dioxan, Halogenkohlenwasserstoffe wie Dichlormethan, Trichlormethan, Tetrachlormethan, 1,2-Dichlorethan, Trichlorethan, Tetrachlorethan, 1,2-Dichlorethan oder Trichlorethylen, Kohlenwasserstoffe wie Benzol, Xylol, Toluol, Hexan, Cyclohexan, oder Erdölfraktionen, Nitromethan, Dimethylformamid, Aceton, Acetonitril oder Hexamethylphosphorsäuretriamid. Ebenso ist es möglich, Gemische der Lösemittel einzusetzen. Besonders bevorzugt sind Tetrahydrofuran, Dimethylformamid, Toluol, Dioxan oder Dimethoxyethan.

Die Reaktion wird im allgemeinen in einem Temperaturbereich von 0°C bis 150°C, bevorzugt von +20°C bis +110°C durchgeführt.

Die Umsetzung kann bei normalen, erhöhtem oder bei erniedrigtem Druck durchgeführt werden (z.B. 0,5 bis 5 bar). Im allgemeinen arbeitet man bei Normaldruck.

Als Palladiumverbindungen im Rahmen der vorliegenden Erfidung eignen sich im allgemeinen PdCl₂(P(C₆H₅)₃)₂, Palladium-bis-dibenzylidenaceton (Pd(dba)₂), [1,1'-Bis-(diphenylphosphino)ferrocen]-Palladium(II)-chlorid (Pd(dppf)Cl₂) oder Pd(P(C₆H₅)₃)₄. Bevorzugt ist Pd(P(C₆H₅)₃)₄.

Als Lösemittel für das Verfahren [C3] eignen sich einige der oben aufgeführten Lösemittel, wobei Benzol besonders bevorzugt ist.

Als Metallsalze eignen sich im Rahmen der Erfindung Kupfersalze oder Rhodium-(II)salze wie beispielsweise CuOTf, Cu(acac)₂, Rh(OAc)₂. Bevorzugt ist Kupferacetylacetonat.

Die Salze werden in katalytischen Mengen eingesetzt.

Die Reaktion wird im allgemeinen in einem Temperaturbereich von 0°C bis 150°C, bevorzugt von +20°C bis +110°C durchgeführt.

Die Umsetzung kann bei normalen, erhöhtem oder bei erniedrigtem Druck durchgeführt werden (z.B. 0,5 bis 5 bar). Im allgemeinen arbeitet man bei Normaldruck.

Das erfindungsgemäße Verfahren [D3] erfolgt mit einer der oben aufgeführten cyclischen Aminbasen, vorzugsweise mit N-Methylpyrrilidon, in einem Temperaturbereich von 100°C bis 200°C, vorzugsweise bei 150°C.

Das erfindungsgemäße Verfahren [E3] erfolgt in einem Temperaturbereich von 150°C bis 210°C, vorzugsweise bei 195°C.

Das erfindungsgemäße Verfahren [F3] erfolgt im allgemeinen in einem der oben aufgeführten Ether, vorzugsweise in Tetrahydrofuran bei Rückflußtemperatur.

Die Umsetzung der freien Methylhydroxygruppe zu den entsprechenden Methylalkoxyverbindungen erfolgt nach üblichen Methoden durch Alkylierung mit Alkylhalogeniden, vorzugsweise Alkyliodiden in Anwesenheit einer der oben aufgeführten Basen, vorzugsweise Natriumhydrid.

Die Verbindungen der allgemeinen Formeln (III-III), (III-V), (III-VI), (III-VII), (III-VIII), (III-IX), (III-XI), (III-XII), (III-XIV), (III-XVI) und (III-XVIII) sind an sich bekannt oder nach üblichen Methoden herstellbar.

Die Verbindungen der allgemeinen Formel (III-II) sind teilweise bekannt und können hergestellt werden, indem man Verbindungen der allgemeinen Formel (III-XXXIX) 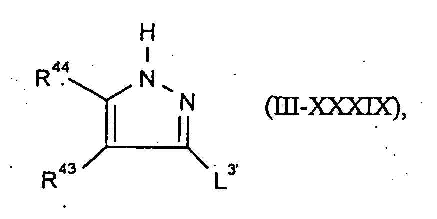 in welcher
R⁴³ und R⁴⁴ die oben angegebene Bedeutung haben
und
- L^{3'}: die oben angegebene Bedeutung von L³ hat und mit dieser gleich oder verschieden ist,
mit Verbindungen der allgemeinen Formel (III-V) in Analogie zu dem oben aufgeführten Verfahren [B3] umsetzt.

Die Verbindungen der allgemeinen Formel (III-IV) sind teilweise bekannt oder im Fall der Stannyle neu und können beispielsweise hergestellt werden, indem man die Verbindungen der allgemeinen Formel (III-IVa) 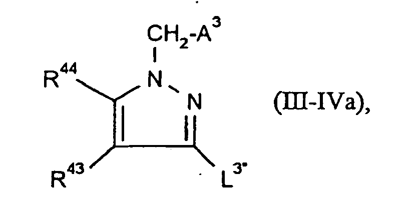 in welcher
R⁴³, R⁴⁴ und A³ die oben angegebene Bedeutung haben,
- L^{3"}: für Triflat oder Halogen, vorzugsweise für Iod steht,
mit Verbindungen der allgemeinen Formel (III-XXX)

(SnR⁵⁵R⁵⁶R⁵⁷)₂ (III-XXX),

in welcher
R⁵⁵, R⁵⁶, R⁵⁷ die oben angegebene Bedeutung haben
wie oben beschrieben Palladiumkatalysiert umsetzt.

Die Verbindungen der allgemeinen Formeln (III-IVa) und (III-XXX) sind bekannt oder nach üblichen Methoden herstellbar.

Die Verbindungen der allgemeinen Formeln (III-X), (III-XIII), (III-XVII) und (III-XIX) sind teilweise neu und können beispielsweise wie oben beschrieben hergestellt werden.

Das Verfahren [H3] verläuft im Rahmen der Erfindung nach üblichen Methoden, insbesondere nach den Ausführungen aus den Publikationen P. Wipf, CP. Miller, J. Org. Chem. 1993, 58, 3604, C.S. Moody et al, Synlett 1996, Seite 825.

Die Verbindungen der allgemeinen Formel (III-XX) sind teilweise bekannt oder aus den entsprechenden Amiden durch Umsetzung mit α-Diazo-β-ketoestern unter Rhodiumsalz-Katalyse herstellbar (vgl. hierzu C.J. Moody et al, Synlett 1996, 825).

Das Verfahren [13] erfolgt nach den üblichen Methoden zur Herstellung von Acetalen. Die Reduktionsschritte werden im weiteren detailliert beschrieben.

Die Verbindungen der allgemeinen Formeln (III-XXI), (III-XXII) und (III-XXIII) sind teilweise bekannt oder als Species neu und können dann wie oben beschrieben hergestellt werden.

Das Verfahren [13] erfolgt in Analogie zu den Publikationen S. Chim u. H.J. Shirie, J. Heterocycl. Chem. 1989, 26, 125 und J. Med. Chem. 1990, 33, 113.

Die Verbindungen der allgemeinen Formeln (III-XXIV) und (III-XXV) sind teilweise bekannt oder nach üblichen Methoden herstellbar.

Die Verbindungen der allgemeinen Formel (III-XXVI) sind teilweise bekannt oder neu und können dann aus den entsprechenden Cyano-substituierten Verbindungen und Hydroxylaminhydrochlorid hergestellt werden. Gegebenenfalls kann dabei eine Base, vorzugsweise Natriummethanolat in Methanol, zugesetzt werden.

Die Verbindungen der allgemeinen Formel (III-XXVII) sind an sich bekannt oder nach üblichen Methoden herstellbar.

Die Verfahren [H3] bis [J3] verlaufen im allgemeinen in einem Temperaturbereich von 0°C bis zur jeweiligen Rückflußtemperatur und Normaldruck.

Die Reduktionen werden im allgemeinen mit Reduktionsmitteln, bevorzugt mit solchen, die für die Reduktion von Carbonyl zu Hydroxyverbindungen geeignet sind, durchgeführt werden. Besonders geeignet ist hierbei die Reduktion mit Metallhydriden oder komplexen Metallhydriden in inerten Lösemitteln, gegebenenfalls in Anwesenheit eines Trialkylborans. Bevorzugt wird die Reduktion mit komplexen Metallhydriden wie beispielsweise Lithiumboranat, Natriumboranat, Kaliumboranat, Zinkboranat, Lithium-trialkylhydrido-boranat, Diisobutylaluminiumhydrid oder Lithiumaluminiumhydrid durchgeführt. Ganz besonders bevorzugt wird die Reduktion mit Diisobutylaluminiumhydrid und Natriumborhydrid durchgeführt.

Das Reduktionsmittel wird im allgemeinen in einer Menge von 1 mol bis 6 mol, bevorzugt von 1 mol bis 4 mol bezogen auf 1 mol der zu reduzierenden Verbindungen, eingesetzt.

Die Reduktion verläuft im allgemeinen in einem Temperaturbereich von -78°C bis +50°C, bevorzugt von -78°C bis 0°C, im Falle des DIBAH, 0°C, Raumtemperatur im Falle des NaBH₄, besonders bevorzugt bei -78°C, jeweils in Abhängigkeit von der Wahl des Reduktionsmittels sowie Lösemittel.

Die Reduktion verläuft im allgemeinen bei Normaldruck, es ist aber auch möglich bei erhöhtem oder erniedrigtem Druck zu arbeiten.

Die Abspaltung der Schutzgruppe erfolgt im allgemeinen in einem der oben aufgeführten Alkohole und/oder THF oder Aceton, vorzugsweise Methanol / THF in Anwesenheit von Salzsäure oder Trifluoressigsäure oder Toluolsulfonsäure in einem Temperaturbereich von 0°C bis 70°C, vorzugsweise bei Raumtemperatur und Normaldruck.

Im Fall, daß die Reste der Formeln -S(O)_{c3}NR⁵⁰R⁵¹ und -S(O)_{c3'}NR⁵⁰R^{51'} vorliegen, werden die entsprechenden unsubstituierten Verbindungen zunächst mit Thionylchlorid umgesetzt. In einem weiteren Schritt erfolgt die Umsetzung mit den Aminen in einem der oben aufgeführten Ethern, vorzugsweise Dioxan. Im Fall c3 = 2 wird anschließend eine Oxidation nach üblichen Methoden durchgeführt. Die Umsetzungen erfolgen in einem Temperaturbereich von 0°C bis 70°C und Normaldruck.

Erfindungsgemäße Verbindungen gemäß Ausführungsform III, in denen R⁴² einen Oxazolyl-Rest der Formel (III-XXVIII) 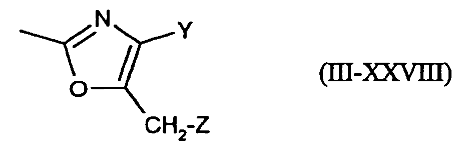 darstellt, worin Y und Z die unten angegebene Bedeutung aufweisen, lassen sich bevorzugt nach dem im folgenden beschriebenen, neuen, allgemein zur Herstellung von Oxazolylverbindungen diesen Typs anwendbarem Verfahren herstellen.

Gegenstand der Erfindung ist somit weiterhin ein Verfahren zur Herstellung von Oxazolylverbindungen der allgemeinen Formel (III-XXIX) 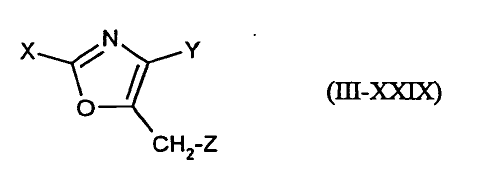 in der
- X und Y: gleich oder verschieden und für gegebenenfalls substituierte aliphatische, cycloaliphatische, araliphatische, aromatische und heterocyclische Reste, einschließlich gesättigter, ungesättigter oder aromatischer heteromono- oder heteropolycyclischer Reste, Carboxyl, Acyl, Alkoxy, Alkoxycarbonyl, Cyano oder für Wasserstoff stehen können,
worin die aromatischen und heterocyclischen Reste durch einen oder mehrere Substituenten substituiert sein können, die aus der Gruppe ausgewählt sind, die besteht aus:
Halogen, Formyl, Acyl, Carboxyl, Hydroxy, Alkoxy, Aroxy, Acyloxy, gegebenenfalls alkylsubstituiertem Amino, Acylamino, Aminocarbonyl, Alkoxycarbonyl, Nitro, Cyano, Phenyl, und
Alkyl, das durch einen oder mehrere Substituenten substituiert sein kann, die aus der Gruppe ausgewählt sind, die besteht aus:
Halogen, Hydroxy, Amino, Carboxy, Acyl, Alkoxy, Alkoxycarbonyl,
   sowie Heterocyclyl und Phenyl, die durch einen oder mehrere Substituenten substituiert sein können, ausgewählt aus:
   Amino, Mercaptyl, Hydroxy, Formyl, Carboxyl, Acyl, Alkylthio, Alkyloxyacyl, Alkoxy, Alkoxycarbonyl, Nitro, Cyano, Trifluormethyl, Azido, Halogen, Phenyl, gegebenenfalls durch Hydroxy, Carboxyl, Acyl, Alkoxy oder Alkoxycarbonyl substituiertem Alkyl,
und worin die aliphatischen, cycloaliphatischen und araliphatischen Reste durch einen oder mehrere Substituenten substituiert sein können, die aus der Gruppe ausgewählt sind, die besteht aus: Fluor, Hydroxy, Alkoxy, Aroxy, Acyloxy, alkylsubstituiertem Amino, Acylamino, Aminocarbonyl, Alkoxycarbonyl und Acyl.

Z ausgewählt wird aus der Gruppe, die besteht aus:
Hydroxy, Alkoxy, gegebenenfalls alkyl- und/oder halogensubstituiertem Arylalkoxy, gegebenenfalls alkyl- und/oder halogensubstituiertes Aroxy, Aroyloxy, Acyloxy, Alkylthio, gegebenenfalls alkyl- und/oder halogensubstituiertes Arylthio, Diacylimido oder einer Gruppe der Formel (III-XXX) 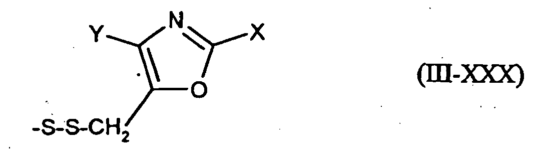 in der Y und X die oben angegebene Bedeutung besitzen,
**dadurch gekennzeichnet, daß** Amide der Formel (III-XXXI) 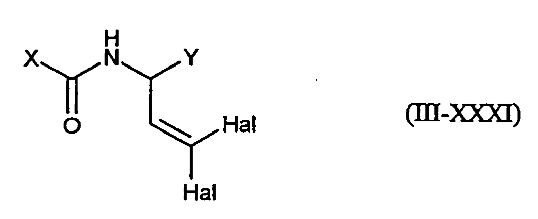 in der Y und X die oben angegebene Bedeutung besitzen und Hal für Chlor oder Brom steht, mit Verbindungen der Formel M1⁺Z⁻ oder M2²⁺(Z⁻)₂ umgesetzt werden, in denen M1 ein Alkalimetall ist, M2 ein Erdalkalimetall ist und Z wie oben definiert ist.

Bezüglich konkreter Beispiele, die die obigen Definitionen der Substituenten in ihrem Umfang enthalten, sei auf die entsprechenden Bedeutungen in den oben gemachten Erläuterungen zu den Verbindungen der Ausführungsform III der vorliegenden Erfindung verwiesen.

In einer bevorzugten Ausführungsform dieses Verfahrens werden Oxazolylverbindungen der vorliegenden Erfindung hergestellt, in denen X in der obigen allgemeinen Formel (III-XXIX) 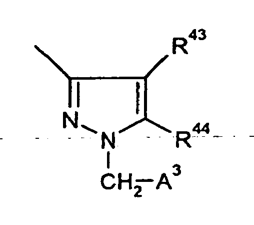 ist, worin R⁴³, R⁴⁴ und A³ wie oben definiert sind und Y Alkyl oder gegebenenfalls alkyl- oder halogensubstituiertes Phenyl ist.

Als Oxazole, die nach dem Herstellungsverfahren erhalten werden, seien beispielsweise genannt: 2,4-Dimethyl-5-methoxymethyl-oxazol, 2-Ethyl-5-methoxymethyl-oxazol, 2-Isopropyl-4-ethyl-5-ethoxymethyl-oxazol, 2-Cyclopropyl-4-hexyl-5-iso-propoxymethyl-oxazol, 2-Phenyl-4-methyl-5-methoxymethyl-oxazol, 2-(m-Trifluormethylphenyl)-4-methyl-5-butoxymethyl-oxazol, 4-Methyl-5-methoxymethyl-2-(m-trifluorphenyl)-oxazol, 2-Phenyl-4-methyl-5-phenoxymethyl-oxazol, 2-(2-Chlor-6-fluor-phenyl)-4-methyl-5-p-tert.-butylphenoxymethyl-oxazol, 2,4-Dimethyl-5-acetoxymethyl-oxazol, 2,4-Dimethyl-5-(3-heptylcarbonyloxy)-methyl-oxazol, 2-Phenyl-4-methyl-5-acetoxymethyl-oxazol, 2-(1-Benzylindazol-3-yl)-5-hydroxymethyl-4-methyl-oxazol, 5-Acetoxymethyl-2-(1-Benzylindazol-3-yl)-4-methyl-oxazol, 2-(1-Benzylindazol-3-yl)-5-methoxymethyl-4-methyl-oxazol, 2-[1-(2-Fluoro-benzyl)indazol-3-yl]-5-hydroxymethyl-4-methyl-oxazol, 2-[1-(2-Fluorobenzyl)-indazol-3-yl]-5-methoxymethyl-4-methyl-oxazol, 2-[1-(2-Fluorobenzyl)indazol-3-yl]-4-methyl-5-(N-phthalimidomethyl)-oxazol, 4-Ethyl-2-[1-(2-Fluorobenzyl)-indazol-3-yl]-5-hydroxymethyl-oxazol, 2-Phenyl-4-ethyl-5-benzoyloxymethyl-oxazol, 2-Phenyl-4-methyl-5-methylmercaptomethyl-oxazol, Bis-[(2-phenyl-4-methyl-oxazol-5-yl)methyl]-disulfid und 2-Phenyl-4-methyl-5-N-phthalimido-methyl-oxazol.

Das erfindungsgemäße Verfahren zur Herstellung der Oxazol-Verbindungen wird z.B. so durchgeführt, daß Amide gemäß Gleichung (a) mit Verbindungen der Formel M1⁺Z⁻ oder M2²⁺(Z⁻)₂ umgesetzt werden: 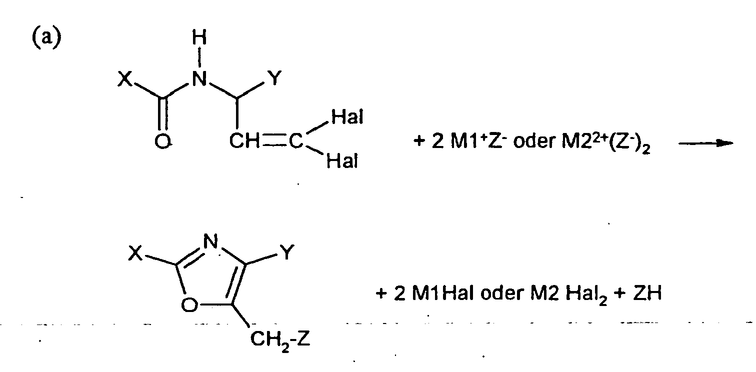 M1 in der Verbindung M1⁺Z⁻ ist ein Alkalimetall ausgewählt aus z.B. Lithium (Li), Natrium (Na) oder Kalium (K), bevorzugt Natrium oder Kalium. Als beispielhafte Verbindungen der Formel M1⁺Z⁻ seien genannt Alkoholate wie Na-methylat, Na-butylat, K-tert.-butylat, Phenolate wie Na-phenolat und Na-4-tert.-butyl-phenolat, Carbonsäuresalze wie Na- oder K-acetat, Li-butyrat, Na-benzoat und Na-2,6-difluorbenzoat, Phthalimid-Salze wie K- und Na-phthalimide, Hydroxide wie KOH, NaOH und LiOH, Mercaptide wie die Natriumsalze von Methylmercaptan oder Thiophenol und Na₂S₂, das zu dem Disulfid der Formel 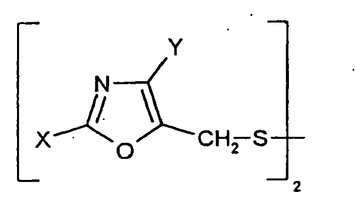 führt.

M2 in der Verbindung M2²⁺(Z⁻)₂ ist ein Erdalkalimetall ausgewählt z.B. aus Magnesium oder Calcium.

Die erfindungsgemäße Umsetzung gemäß Gleichung (a) wird in Lösungsmitteln bei Temperaturen von etwa 20°C bis 200°C ausgeführt. Als Lösungsmittel eignen sich polare Verbindungen wie z.B. Dimethylformamid, Dimethylacetamid, N-Methylpyrrolidon, N-Methyl-ε-caprolactam und Dimethylsulfoxid, weiterhin kommen auch Verbindungen der Formel Z-H als Lösungsmittel infrage, beispielsweise kann die Umsetzung der Amide mit Na-methylat mit Erfolg in Methanol ausgeführt werden. Zusatz von basischen Hilfsstoffen wie z.B. K₂CO₃ oder Cs₂CO₃ kann vorteilhaft sein. Die Isolierung der erhaltenen Oxazole gelingt nach Entfernung unlöslicher Salze durch Filtration und gegebenenfalls destillativer Abtrennung von Lösungsmitteln durch Extraktion der Oxazole mit geeigneten Lösungsmitteln, wie z.B. Kohlenwasserstoffen wie Cyclohexan oder Toluol oder Chlorkohlenwasserstoffen wie z.B. Dichlormethan oder Chlorbenzol oder Estern wie Essigester oder Ethern aus dem zur Abtrennung von wasserlöslichen Produkten mit Wasser versetztem Rohprodukt. Die Reinigung des Rohproduktes kann durch übliche Verfahren wie z.B. Destillation oder Kristallisation oder chromatographisch ausgeführt werden.

Die Amide als Ausgangsverbindungen werden nach bekannten Verfahren, z.B. ausgehend von Verbindungen der Formel a, b oder c erhalten.

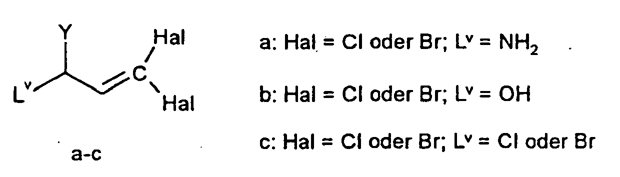

Ausgehend von Aminen der Formel a werden Amine der Formel (XXXI) in bekannter Weise durch Umsetzung mit entsprechenden Acylierungsmitteln, wie z.B. Säurehalogeniden, Estern oder Säuren, erhalten.

Ausgehend von Verbindungen der Formel b oder c erhält man Amide in bekannter Weise durch Umsetzung mit Nitrilen in Gegenwart von starken Säuren.

Amide entsprechend der Formel a sind z.B. durch Hydrolyse unter sauren Bedingungen aus Amiden zugänglich, die in bekannter Weise durch Ritter-Reaktion aus Alkylhalogeniden bzw. Allylalkoholen der Formel b und c erhalten werden. Schließlich können solche Amine auch über allylische nucleophile Substitution mit z.B. Phthalimidsalzen aus den entsprechenden Allylhalogeniden der Formel c über die Stufe der entsprechende substituierten Phthalimide und nachfolgende Solvolyse erhalten werden.

Verbindungen der Formel b sind gemäß Gleichung (b) und (c) in zwei Reaktionsschritten aus einfachen Ausgangsmaterialien in bekannter Weise leicht zugänglich:

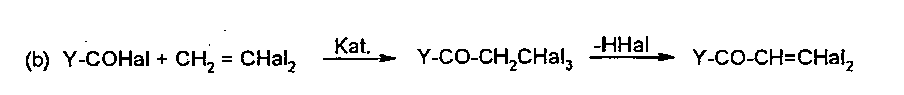

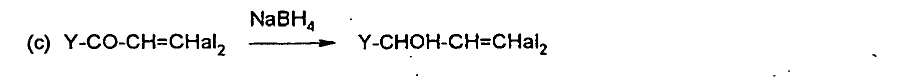

Verbindungen der Formel c werden in bekannter Weise z.B. durch radikalisch initiierte Addition von Tetrachlor- oder Tetrabrommethan an entsprechende olefinische Verbindungen und nachfolgende Eliminierung von Halogenwasserstoff gemäß Gleichung (d) erhalten:

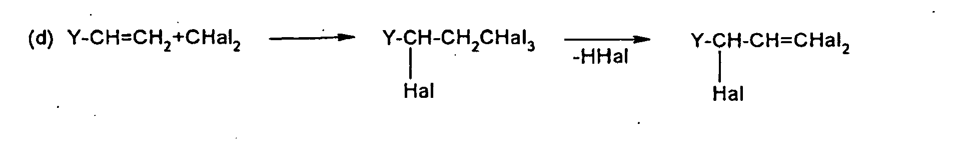

Darüber hinaus umfaßt die Erfindung die Kombination der erfindungsgemäßen Verbindungen der allgemeinen Formeln (III-I)/(III-Ia) mit organischen Nitraten und NO-Donatoren.

Organische Nitrate und NO-Donatoren im Rahmen der Erfindung sind im allgemeinen Substanzen, die über die Freisetzung von NO bzw. NO-Species ihre therapeutische Wirkung entfalten. Bevorzugt sind Natriumnitroprussid, Nitroglycerin, Isosorbiddinitrat, Isosorbidmononitrat, Molsidomin und SIN-1.

Außerdem umfaßt die Erfindung die Kombination mit Verbindungen, die den Abbau von cyclischen Guanosinmonophosphat (cGMP) inhibieren. Dies sind insbesondere Inhibitoren der Phosphodiesterasen 1, 2 und 5; Nomenklatur nach Beavo und Reifsnyder (1990) TIPS 11 S. 150 - 155. Durch diese Inhibitoren wird die Wirkung der erfindungsgemäßen Verbindungen potenziert und der gewünschte pharmakologische Effekt gesteigert.

Die erfindungsgemäßen Verbindungen der allgemeinen Formeln (III-I)-(III-Id) zeigen ein nicht vorhersehbares, wertvolles pharmakologisches Wirkspektrum.

Die erfindungsgemäßen Verbindungen der allgemeinen Formeln (III-I)-(III-Id) führen zu einer Gefäßrelaxation/Thrombozytenaggregationshemmung und zu einer Blutdrucksenkung sowie zu einer Steigerung des koronaren Blutflusses. Diese Wirkungen sind über eine direkte Stimulation der löslichen Guanylatzyklase und einem intrazellulären cGMP-Anstieg vermittelt. Außerdem verstärken die erfindungsgemäßen Verbindungen die Wirkung von Substanzen, die den cGMP-Spiegel steigern, wie beispielsweise EDRF (Endothelium derived relaxing factor), NO-Donatoren, Protoporphyrin IX, Arachidonsäure oder Phenylhydrazinderivate.

Sie können daher in Arzneimitteln zur Behandlung von kardiovaskulären Erkrankungen wie beispielsweise zur Behandlung des Bluthochdrucks und der Herzinsuffizienz, stabiler und instabiler Angina pectoris, peripheren und kardialen Gefäßerkrankungen, von Arrhythmien, zur Behandlung von thromboembolischen Erkrankungen und Ischämien wie Myokardinfarkt, Hirnschlag, transistorisch und ischämische Attacken, periphere Durchblutungsstörungen, Verhinderung von Restenosen wie nach Thrombolysetherapien, percutan transluminalen Angioplastien (PTA), percutan transluminalen Koronarangioplastien (PTCA), Bypass sowie zur Behandlung von Arteriosklerose und Krankheiten des Urogenitalsystems wie beispielsweise Prostatahypertrophie, erektile Dysfunktion und Inkontinenz eingesetzt werden.

Zur Feststellung der kardiovaskulären Wirkungen wurden folgende Untersuchungen durchgeführt: In in vitro-Untersuchungen an Zellen vaskulären Ursprungs wurde der Einfluß auf die Guanylatzyklase-abhängige cGMP-Bildung mit und ohne NO-Donor geprüft. Die antiaggregatorischen Eigenschaften wurden an mit kollagenstimulierten menschlichen Thrombozyten gezeigt. Die gefäßrelaxierende Wirkung wurde an mit Phenylephrin vorkontrahierten Kaninchenaortenringen bestimmt. Die blutdrucksenkende Wirkung wurde an narkotisierten Ratten untersucht.

### Stimulation der löslichen Guanylatzyklase in primären Endothelzellen

Primäre Endothelzellen wurden aus Schweineaorten durch Behandlung mit Kollagenase-Lsg. isoliert. Anschließend wurden die Zellen in Kulturmedium bis zum Erreichen der Konfluenz kultiviert. Für die Untersuchungen wurden die Zellen passagiert, in Zellkulturplatten ausgesät und bis zum Erreichen der Konfluenz subkultiviert. Zur Stimulation der endothelialen Guanylatzyklase wurde das Kulturmedium abgesaugt und die Zellen einmal mit Ringerlösung gewaschen und in Stimulationspuffer mit oder ohne NO-Donor (Natrium-Nitroprussid, SNP, 1 µM) inkubiert. Im Anschluß daran wurden die Testsubstanzen (Endkonzentration 1 µM) zu den Zellen pipettiert. Nach Ende der 10-minütigen Inkubationszeit wurde die Pufferlösung abgesaugt und die Zellen 16 Stunden lang bei -20°C lysiert. Anschließend wurde das intrazelluläre cGMP radioimmunologisch bestimmt.

**Tabelle A**

| Beispiel Nr. | cGMP-Steigerung % |
|---|---|
| III-71 | 315 |
| III-73 | >1000 |
| III-74 | 114 |
| III-75 | >1000 |
| III-76 | 397 |
| III-77 | >1000 |
| III-78 | 223 |
| III-79 | 124 |
| III-80 | >1000 |
| III-81 | 110 |
| III-82 | 455 |
| III-87 | 268 |
| III-91 | 479 |
| III-92 | 319 |
| III-93 | 271 |

### Gefäßrelaxierende Wirkung in vitro

1,5 mm breite Ringe einer isolierten Kaninchen-Aorta werden einzeln unter einer Vorspannung in 5 ml-Organbäder mit 37°C warmer, carbogenbegaster Krebs-Henseleit-Lösung gebracht. Die Kontraktionskraft wird verstärkt und digitalisiert sowie parallel auf Linienschreiber registriert. Zur Erzeugung einer Kontraktion wird Phenylephrin dem Bad kumulativ in ansteigender Konzentration zugesetzt.

Nach mehreren Kontrollzyklen wird die zu untersuchende Substanz in jedem weiteren Durchgang in jeweils steigender Dosierung untersucht und mit der Höhe der im letzten Vordurchgang erreichten Kontraktion verglichen. Daraus wird die Konzentration errechnet, die erforderlich ist, um die Höhe des Kontrollwertes um 50 % zu reduzieren (IC₅₀). Das Standardapplikationsvolumen beträgt 5 µl.

**Tabelle B**

| Beispiel Nr. | Aorta IC₅₀ |
|---|---|
| III-71 | 5 µM |
| III-73 | 9,4 µM |
| III-75 | 2,2 µM |
| III-76 | 7,4 µM |
| III-77 | 8,3 µM |
| III-78 | 10 µM |
| III-79 | 13 µM |
| III-80 | 3,6 µM |
| III-81 | 12 µM |
| III-82 | 15 µM |
| III-87 | 19 µM |
| III-88 | 7,1 µM |
| III-90 | 4,1 µM |
| III-95 | 2,4 µM |

### Blutdruckmessungen an narkotisierten Ratten

Männliche Wistar-Ratten mit einem Körpergewicht von 300 - 350 g werden mit Thiopental (100 mg/kg i.p.) anästhesiert. Nach Tracheotomie wird in die Femoralarterie ein Katheter zur Blutdruckmessung eingeführt. Die zu prüfenden Substanzen werden als Suspension in Tyloselösung mittels Schlundsonde in verschiedenen Dosen oral verabreicht.

### Thrombozytenaggregationshemmung in vitro

Zur Bestimmung der thrombozytenaggregationshemmenden Wirkung wurde Blut von gesunden Probanden beiderlei Geschlechts verwendet. Als Antikoagulans wurden einem Teil 3,8%iger wäßriger Natriumzitratlösung 9 Teile Blut zugemischt. Mittels Zentrifugation erhält man aus diesem Blut plättchenreicheres Zitratplasma (PRP).

Für die Untersuchungen wurden 445 µl PRP und 5 µl der Wirkstofflösung bei 37°C im Wasserbad vorinkubiert. Anschließend wurde die Thrombozytenaggregation nach der turbidometrischen Methode im Aggregometer bei 37°C bestimmt. Hierzu wurde die vorinkubierte Probe mit 50 µl Kollagen, einem aggregationsauslösenden Agens, versetzt, und die Veränderung der optischen Dichte erfaßt. Zur quantitativen Ausweitung wurde der maximale Aggregationsresponse ermittelt und daraus die prozentuale Hemmung gegenüber der Kontrolle errechnet.

**Tabelle D**

| Beispiel Nr. | IC₅₀ (µg/ml) |
|---|---|
| III-71 | 50 |
| III-72 | 1 |

Die in der vorliegenden Erfindung beschriebenen Verbindungen der Ausführungsform III stellen auch Wirkstoffe zur Bekämpfung von Krankheiten im Zentralnervensystem dar, die durch Störungen des NO/cGMP-Systems gekennzeichnet sind. Insbesondere sind sie geeignet zur Beseitigung kognitiver Defizite, zur Verbesserung von Lern- und Gedächtnisleistungen und zur Behandlung der Alzheimer'schen Krankheit. Sie eignen sich auch zur Behandlung von Erkrankungen des Zentralnervensystems wie Angst-, Spannungs- und Depressionszuständen, zentralnervös bedingten Sexualdysfunktionen und Schlafstörungen, sowie zur Regulierung krankhafter Störungen der Nahrungs-, Genuß- und Suchtmittelaufnahme.

Weiterhin eignen sich diese Wirkstoffe auch zur Regulation der cerebralen Durchblutung und stellen somit wirkungsvolle Mittel zur Bekämpfung von Migräne dar.

Auch eignen sie sich zur Prophylaxe und Bekämpfung der Folgen cerebraler Infarktgeschehen (Apoplexia cerebri) wie Schlaganfall, cerebraler Ischämien und des Schädel-Hirn-Traumas. Ebenso können die erfindungsgemäßen Verbindungen zur Bekämpfung von Schmerzzuständen eigesetzt werden.

Zur vorliegenden Erfindung gehören pharmazeutische Zubereitungen, die neben nicht-toxischen, inerten pharmazeutisch geeigneten Trägerstoffen eine oder mehrere erfindungsgemäße Verbindungen enthalten oder die aus einem oder mehreren erfindungsgemäßen Wirkstoffen bestehen, sowie Verfahren zur Herstellung dieser Zubereitungen.

Der oder die Wirkstoffe können gegebenenfalls in einem oder mehreren der oben angegebenen Trägerstoffe auch in mikroverkapselter Form vorliegen.

Die therapeutisch wirksamen Verbindungen sollen in den oben aufgeführten pharmazeutischen Zubereitungen vorzugsweise in einer Konzentration von etwa 0,1 bis 99,5, vorzugsweise von etwa 0,5 bis 95 Gew.-%, der Gesamtmischung vorhanden sein.

Die oben aufgeführten pharmazeutischen Zubereitungen können außer den erfindungsgemäßen Verbindungen auch weitere pharmazeutische Wirkstoffe enthalten.

Im allgemeinen hat es sich sowohl in der Human- als auch in der Veterinärmedizin als vorteilhaft erwiesen, den oder die erfindungsgemäßen Wirkstoffe in Gesamtmengen von etwa 0,5 bis etwa 500, vorzugsweise 5 bis 100 mg/kg Körpergewicht je 24 Stunden, gegebenenfalls in Form mehrerer Einzelgaben, zur Erzielung der gewünschten Ergebnisse zu verabreichen. Eine Einzelgabe enthält den oder die erfindungsgemäßen Wirkstoffe vorzugsweise in Mengen von etwa 1 bis etwa 80, insbesondere 3 bis 30mg/kg Körpergewicht.

### Ausgangsverbindungen

### Abkürzungen:

- Ph =: Phenyl
- Et =: Ethyl
- Me =: Methyl
- EE =: Essigester
- H =: Hexan
- PE =: Petrolether
- MeOH =: Methanol
- E =: Ether
- DMF =: Dimethylformamid
- Ac =: Acetyl
- KOH =: Kaliumhydroxid
- NMP =: N-Methylpyrrolidon

### Ausführungsform III der Erfindung

### Beispiel III/8 A

### 1-Benzyl-3-iodindazol

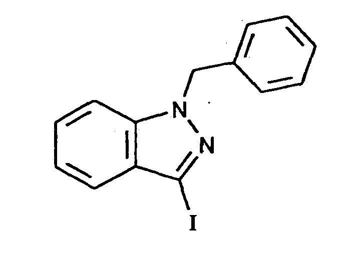

Eine Lösung von 2.99 g Iodindazol (12.25 mmol) in 10 ml THF wurde zu einer Suspension von 515 mg NaH (60% in Öl, 12.88 mmol) in 20 ml THF zugetropft. Nach 15 mn wurden 1.55 ml Benzylbromid zugegeben. Nach 6h bei RT und 3 h bei 40°C wurde die Reaktion mit Wasser versetzt und mit Ether extahiert. Die organischen Phasen wurden mit Natriumsulfat getrocknet und eingeengt. Nach Chromatographie (SiO₂; Petrolether:Essigester 9:1) erhält man 3.351 g eines viskosen Öls, das in vacuo fest wird.
Mp: 51.5-52.5°C.
R_{f} = 0,38 (Hexan Essigester 3:1).

### Beispiel III/9 A

### 1 -Benzyl-3-cyanoindazol

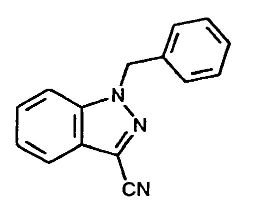

1.0 g 3-Cyanoindazol (7.0 mmol) und 1.7 ml Benzylbromid (14.0 mmol) in 6 ml THF wurden portionsweise mit 420 mg NaH (60% in Öl, 10.3 mmol) versetzt und 15 h bei RT rühren lassen. Die Reaktion wurde mit 2 Tropfen Wasser gequencht, die Mischung eingeengt und chromatographiert (SiO₂; Petrolether:Essigester 3:1). Man erhält 1.3 g eines Feststoffs.
Mp: 91°C.

### Beispiel III/10 A

### 1-Benzyl-3 -trimethylstannyl-indazol:

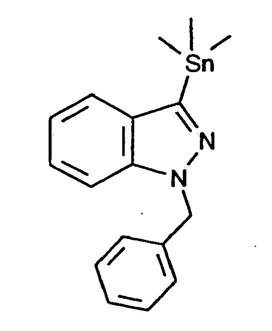

1,67 g 1-Benzyl-3-iodindazol (5,00 mmol), 4,95 g Hexamethyldizinn (15,0 mmol) und 530 mg PD(PPh₃)₄ (10 Mol-%) wurden in 50 ml wasserfreiem 1,4-Dioxan über Nacht unter Rückfluß erhitzt. Die auf Raumtemperatur abgekühlte Mischung wurde mit 15 ml 1M Kaliumfluorid-Lösung und 50 ml Ethylacetat versetzt und 30 Minuten gerührt. Nach Abfiltrieren des Niederschlags wurde die organische Phase des Filtrats mit Wasser gewaschen, über Magnesiumsulfat getrocknet und am Rotationsverdampfer vom Lösungsmittel bereit. Trocknung des Rückstands bei 50°C im Hochvakuum lieferte das Produkt in Form eines weißen Feststoffs, der ohne weitere Reinigung in die anschließenden Pd-katalysierten Kupplungen eingesetzt werden konnte. Ausbeute: 78%
R_{f}: 0,32 (Kieselgel; Cyclohexan/Ethylacetat 16:1)
MS-EI: 372 (Sn, M⁺, 23), 357 (Sn, 56), 207 (100), 165 (Sn, 61), 91 (68).

### Herstellungsbeispiele

### Ausführungsform III der Erfindung

### Beispiel III/69

### 1-Benzyl-3-(1-methyl-imidazol-2-yl)-indazol

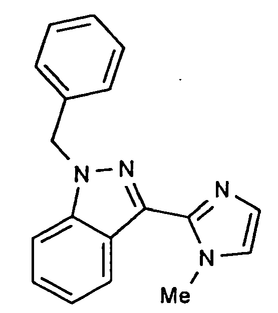

2.50 g 1-Benzyl-3-iodindazol (7.48 mmol), 3.33 g 1-Methyl-2-tributylstannyl-imidazol (8.98 mmol) (K.Gaare, K. Undheim et al, Acta Chem. Scand 1993, 47, 57) und 432 mg Tetrakis-triphenylphosphinpalladium (0.37 mmol) in 10 ml DMF unter Argon wurden 2 Tage bei 80°C erhitzt. Nach dem Abkühlen wurde die Mischung mit Wasser versetzt und mit Essigester extrahiert. Die organischen Phasen wurden mit Natriumsulfat getrocknet und eingeengt. Nach Chromatographie (SiO₂; CH₂Cl₂:MeOH 100:1) erhält man 2.40 g eines Öls.
MS: (Cl, NH₃): 289 (M+H⁺, 100).

### Beispiel III/70

### 2-(1-Benzyl-indazol-3-yl)-oxazol-5-carbonsäure Ethylester

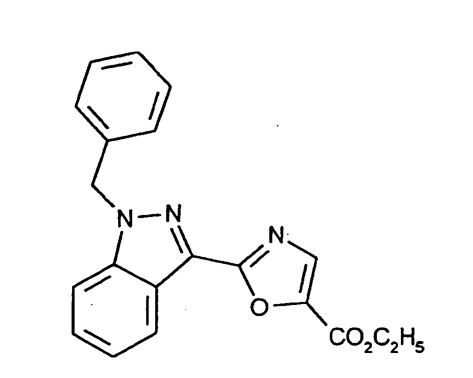

Eine Lösung von Diazobrenztraubensäure Ethylester (250 mg, 1.76 mmol) (T. Ohsumi & H. Neunhofer, Tetrahedron 1992, 48, 5227) in 4 ml Benzol wurde in 4 h zu einer refluxierenden Lösung von 600 mg 1-Benzyl-3-cyanoindazol (2.57 mmol) und 0.8 mg Kupfer(II)acetylacetonat (3 mmol) in 1 ml Benzol zugetropft. Danach wurde die Reaktionsmischung weiter 15 mn auf Rückfluß erhitzt, abgekühlt, und in vacuo abgedampft. Der Rückstand wurde chromatographiert (SiO₂; Cyclohexan:Essigester 3:1). Man erhält 67 mg eines gelben Öls.
R_{f} = 0,11 (Hexan / Essigester 3:1).

### Beispiel III/71

### 1-Benzyl-3-(5-Hydroxymethyl-oxazol-2-yl)-indazol

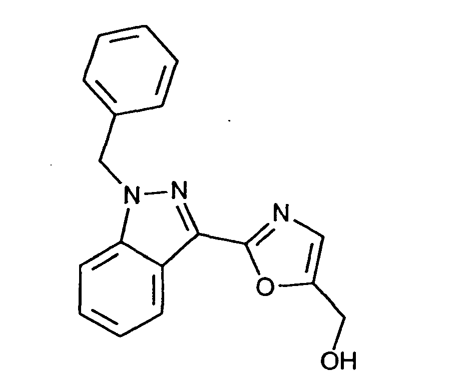

18 mg Lithium-Aluminium-Hydrid (0.47 mmol) wurden zu einer Lösung von 67 mg 2-(1-Benzyl-indazol-3-yl)-oxazol-5-carbonsäure Ethylester in 2 ml Ether bei 0°C zugegeben. Nach 3h bei 0°C wurde die Reaktion weiter 24h bei RT gerührt, dann mit Wasser versetzt und mit Ether 3 mal extrahiert. Die organischen Phasen wurden mit Natriumsulfat getrocknet und eingeengt. Nach Chromatographie (SiO₂; Cyclohexan:Essigester 2:1 bis 3:2) erhält man 12 mg eines weissen Feststoffs.
R_{f} = 0,12 (Hexan / Essigester 1:1).

### Beispiel III//72

### 2-(1-Benzyl-indazol-3-yl)-thiazol-4-carbonsäure Ethylester

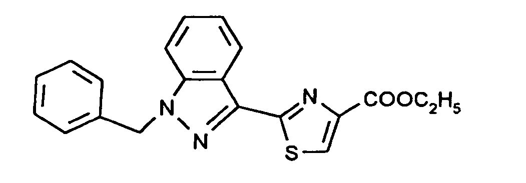

148 mg 1-Benzyl-3-trimethylstannyl-indazol (0.399 mmol), 86 mg 2-Bromthiazol-4-carbonsäure Ethylester (0.364 mmol) (Erlenmeyer et al. Helv. Chim. Acta 1942 (25) 1073) und 42 mg Pd(PPh₃)₄ wurden in 2 ml DMF unter Argon 2 Tage bei 80°C gerührt. Nach dem Abkühlen wurde die Mischung mit Wasser versetzt und mit Essigester extrahiert. Die organischen Phasen wurden mit Natriumsulfat getrocknet und eingeengt. Nach Chromatographie (SiO₂; Petrolether:Essigester 3:1) erhält man 75 mg eines weissen Feststoffs (52%).
R_{f}: 0.31 (Hexan:Essigester 3: 1).
Mp: 95-96°C.

In Analogie zu den oben aufgeführten Vorschriften werden die in der Tabelle III/1 aufgeführten Verbindungen hergestellt:

**Tabelle III/1:**

| | | | |
|---|---|---|---|
| 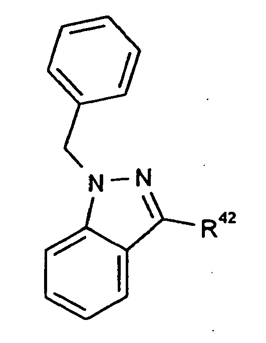 | | | |

| **Bsp.-Nᵣ.** | **R⁴²** | **R_{f} * / LM** | **Ausbeute (% d.Th.)** |
|---|---|---|---|
| III/73 | 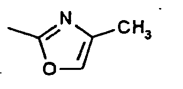 | 0,14 (H:E 3:1) Mp: 77°C | 26 |
| III/74 | 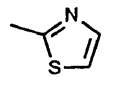 | 0,25 (H:E 3:1) Mp: 77°C | 37 |

| | | | |
|---|---|---|---|
| H: Hexan E: Essigester | | | |

Die Verbindungen, die in der Tabelle III/2 aufgeführt sind, wurden entweder in Analogie zu den oben aufgeführten Vorschriften hergestellt oder über die entsprechenden Indazolderivaten 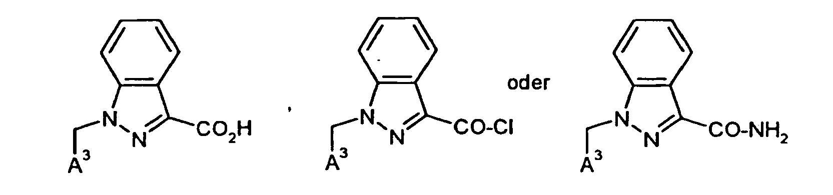 (im folgenden mit Indazol-CO₂H, Indazol-Co-Cl oder Indazol-CO-NH₂ gekennzeichnet)
nach den unter [A3]-[G3] aufgeführten Verfahrensvarianten erhalten.

**Tabelle III/2**

| Bsp. Nr. | Struktur | Herstellungs-methode | Ausbeute/ F°C | R_{f} |
|---|---|---|---|---|
| III/75 | 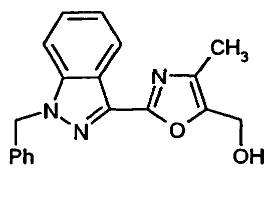 | * [D3] | 65 % aus OAc/ 128°C | 0,17 (CH₂Cl₂ MeOH 100:5) |
| III/76 | 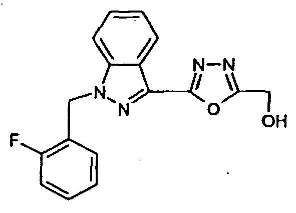 | * [G3] | 86 % aus OAc/ 138°C | 0,12 (CH₂Cl₂ MeOH 100:5) |
| III/77 | 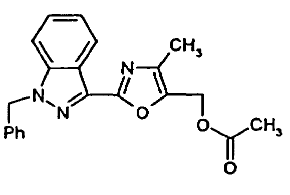 | * [D3] | 24 %/ | 0,15 (H:EE 3:1) |
| III/78 | 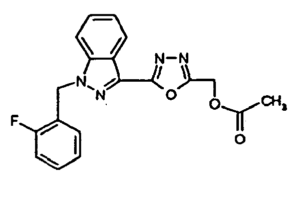 | * [G3] | 92 %/ 107°C | 0,16 (H:EE 3:1) |
| III/79 | 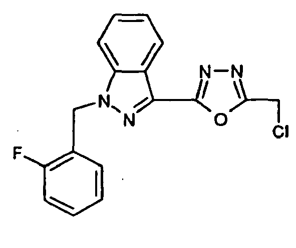 | * [G3] | 52 %/ 111°C | 0,77 (CH₂Cl₂ MeOH 100:5) |
| III/80 | 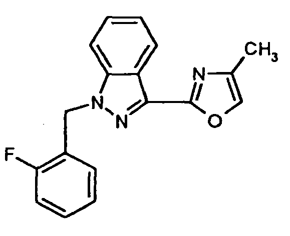 | [B3] | 24 %/ 106°C | 0,22 (H:EE 3:1) |
| III/81 | 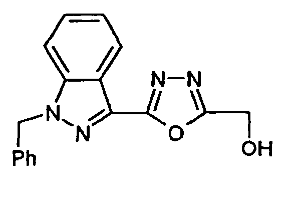 | * [G3] | 50 %/ 153°C | 0,22 (DCM:MeO H 100:5) |
| III/82 | 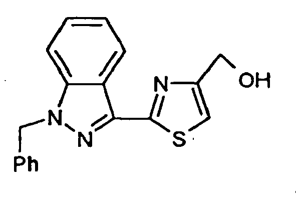 | [B3] | 41 %/ 150°C | 0,11 (H:EE 3:1) |
| III/83 | 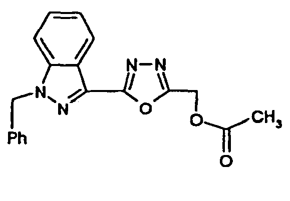 | * [G3] | 35 %/ 135°C | 0,69 (DCM MEOH 100:5) |
| III/84 | 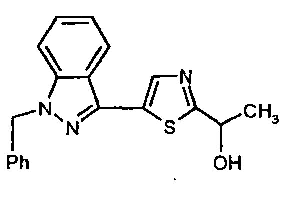 | [A3] [B3] +Entschützen | 40 %/ 94°C | 0,24 (DCM:M 100:5) |
| III/85 | 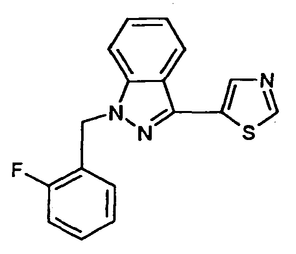 | [A3] | 20 %/ 87°C | 0,43 (DCM:M 100:5) |
| III/86 | 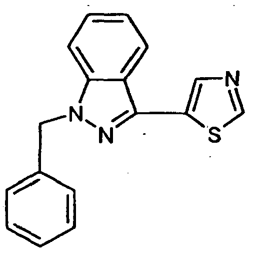 | [A3] | 9 %/ 76°C | 0,44 (DCM:M 100:2) |
| III/87 | 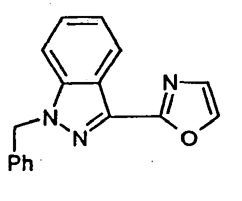 | * [E3] | 22 %/ 80°C | 0,70 (DCM:M 100:5) |
| III/88 | 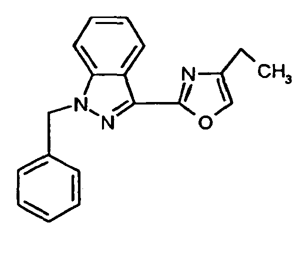 | * [F4] | 5,5 %/ 60°C | 0,63 (H:EE 1:1) (Alox) |
| III/89 | 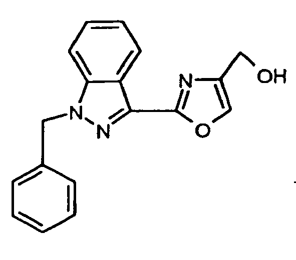 | * [F3] + Red. | 36 %/ 122°C | 0,24 (CH₂Cl₂: CH₃OH 100:1) |
| III/90 | 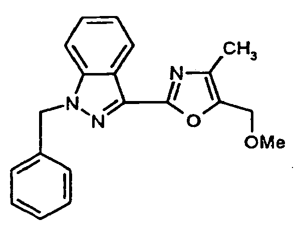 | * [D3] | 40% | 0,08 (H:EE 3:1) |
| III/91 | 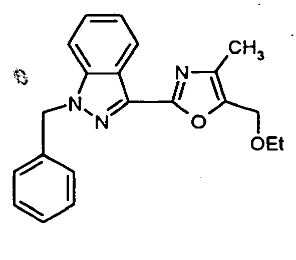 | * [D3] | 44 % | 0,43 (H:EE 1:1) |
| III/92 | 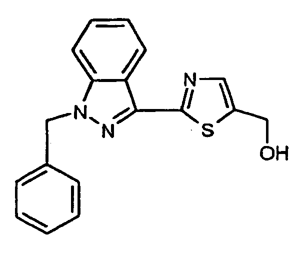 | [B3] +Entschützen | -100% 121°C | 0,09 (CH₂Cl₂: CH₃OH 100:1) |
| III/93 | 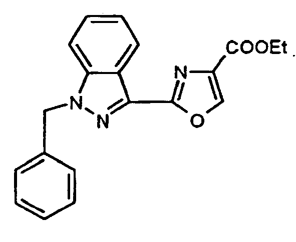 | * [F3] | 19 % | 0,29 (CH₂Cl: CH₃OH 100:1) |
| III/94 | 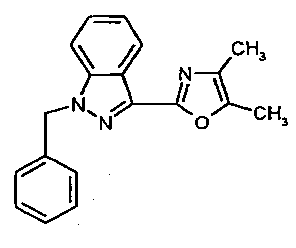 | * [F3] | 14 %/ 109°C | 0,87 (CH₂Cl₂: CH₃OH 100:1) (Alox) |
| III/95 | 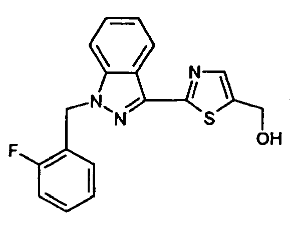 | [B3] +Entschützen | 57 % 139°C | 0,10 (CH₂Cl₂: CH₃OH 100:1) |
| III/96 | 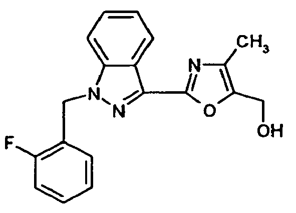 | * [D3] | 59 % 144°C | 0,21 (H:EE 1:1) |
| III/ 97 | 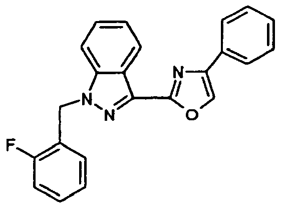 | * [F3] | 11 % | 0,43 (PE:EE 1:1) Alox |
| III/ 98 | 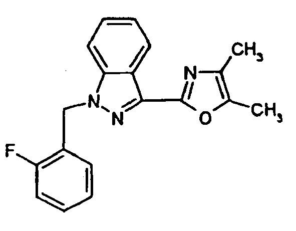 | * [F3] | 13 % 145°C | 0,67 (H:EE 3:1) |
| III/ 99 | 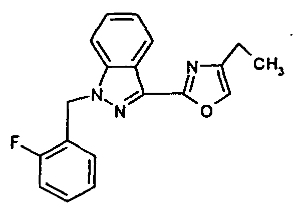 | * [F3] | 16 % 73°C | 0,47 (H:EE 3:1) Alox |

| | | | | |
|---|---|---|---|---|
| * = Aufbau aus Indazol 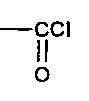 oder Indazol-COOH oder Indazol 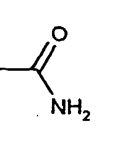 [ ] = siehe Verfahrensschema | | | | |

**Tabelle III/3**

| Bsp.- Nr. | Struktur | Herstellungs-methode | Ausbeute F °C | R_{f} |
|---|---|---|---|---|
| III/ 100 | 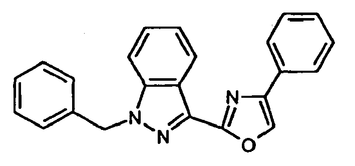 | F3 | 11 % | 0,47 (PE/E 1:1) Al₂O₃ |
| III/ 101 | 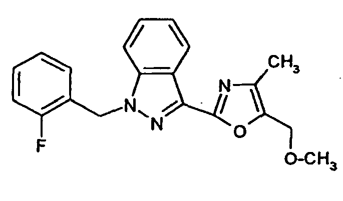 | D3 | 20 % 87°C | 0.18 (100.1) |
| III/ 102 | 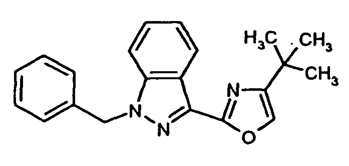 | F3 | 20 % | 0,26 (PE/E 5:1) Al₂O₃ |
| III/ 103 | 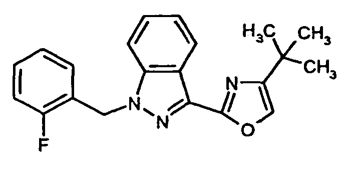 | F3 | 8,5 % 75°C | 0,27 (Hex/EE 1:1) |
| III/ 104 | 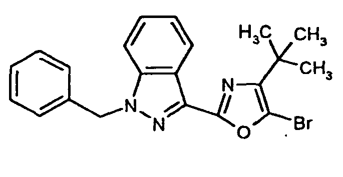 | F3 Nebenprodukt | 9,5 | 0,368 (PE/E 5:1) Al₂O₃ |
| III/ 105 | 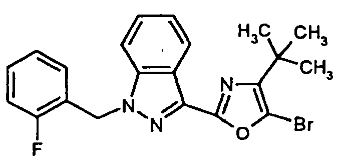 | F3 Nebenprodukt | 12 % | 0,381 (PE/E 5:1) Al₂O₃ |
| III/ 106 | 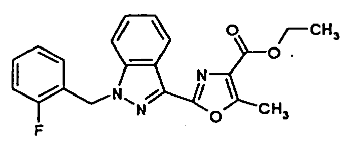 | H3 | 75 % 188°C | 0,21 (Hex/EE 3:1) Al₂O₃ |
| III/ 107 | 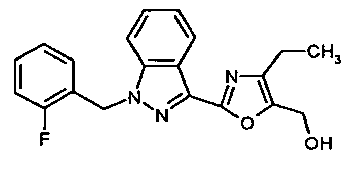 | D3 | 52 % 138°C | 0,09 (Cyclo/ EE 2:1) Al₂O₃ |
| III/ 108 | 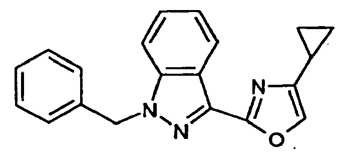 | F3 | 11 % 71°C | 0,486 (PE/E 1:1) Al₂O₃ |
| III/ 109 | 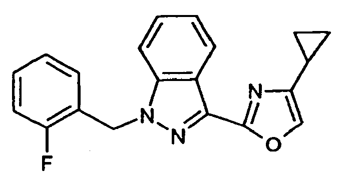 | F3 | 10% | 0,625 (PE/E 1:1) Al₂O₃ |
| III/ 110 | 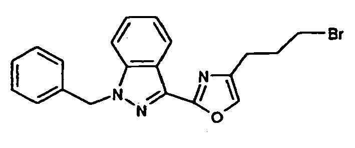 | F3 Nebenprodukt | 12 % 72°C | 0,399 (PE/E 1:1) Al₂O₃ |
| III/ 111 | 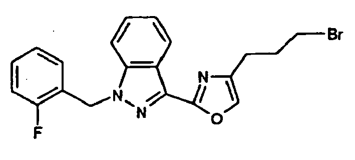 | F3 Nebenprodukt | 8.5 % | 0,581 (PE/E 1:1) Al₂O₃ |
| III/ 112 | 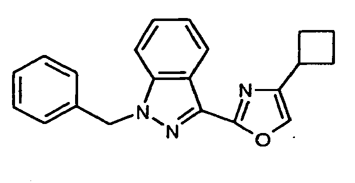 | F3 | 15 % 86°C | 0,417 (PE/E 1:1) Al₂O₃ |
| III/ 113 | 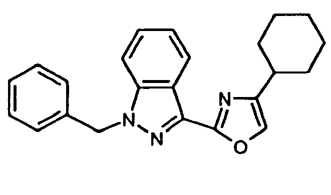 | F3 | 7 % 88°C | 0,622 (PE/E 1:1) Al₂O₃ |
| III/ 114 | 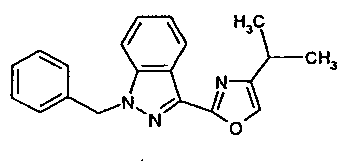 | F3 | 14 % 54°C | 0,667 (PE/E 1:1) Al₂O₃ |
| III/ 115 | 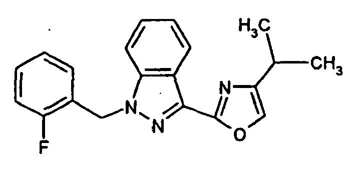 | F3 | 13 % 68°C | 0,667 (PE/E 1:1) Al₂O₃ |
| III/ 116 | 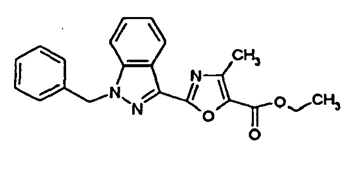 | F3 mit 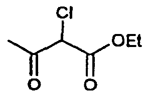 | 27 % 121°C | 0,453 (E/PE 1:1) Al₂O₃ |
| III/ 117 | 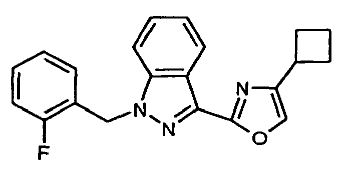 | F3 | 4 % | 0,331 (E/PE 1:3) Al₂O₃ |
| III/ 118 | 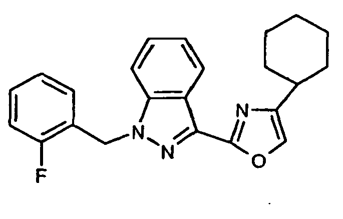 | F3 | 7 % | 0,674 (PE/E 1:1) Al₂O₃ |
| III/ 119 | 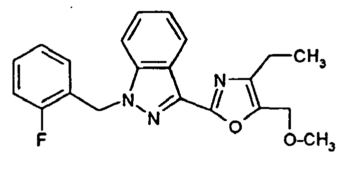 | D3 + Alkylierung | 53 % 101°C | 0,5 (Cyclo/ EE 2: 1) Al₂O₃) |
| III/ 120 | 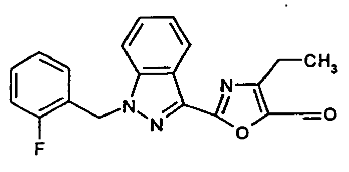 | D3 + Oxidation | 35 % 121°C | 0,446 (Cyclo/ EE 2:1) Al₂O₃ |
| III/ 121 | 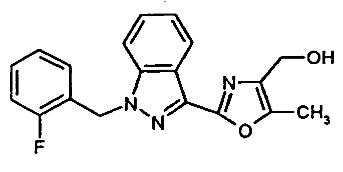 | H3 + Reduktion | 63 % 142°C | 0,037 (Hex/EE 3:1) |
| III/ 122 | 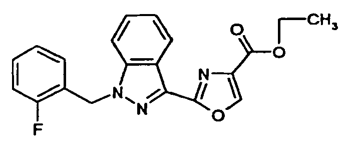 | F3 | 10% | 0,55 (Cycl/ EE 2:1 |
| III/ 123 | 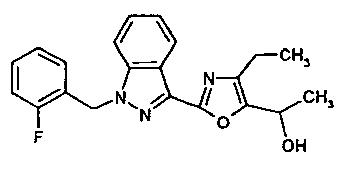 | D3 + Oxidation + Addition | 80 % 81°C | 0,086 (Hex/EE 3:1) |
| III/ 124 | 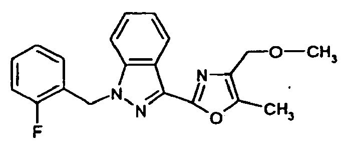 | H3 + Reduktion + Alkylation | 97 % 91°C | 0,515 (Hex/EE 1:1) |
| III/ 125 | 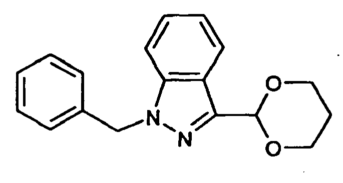 | 13 | 86 % 111°C | 0,162 (Hex/EE 3:1) |
| III/ 126 | 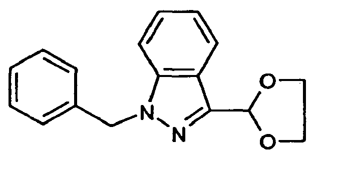 | 13 | 92% 83°C | 0,179 (Hex/EE 3:1) |
| III/ 127 | 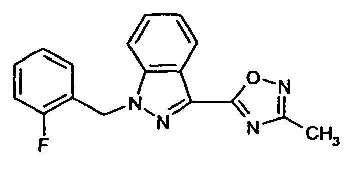 | J3 | 38 % 173°C | 0,30 (H:EE 3:1) |
| III/ 128 | 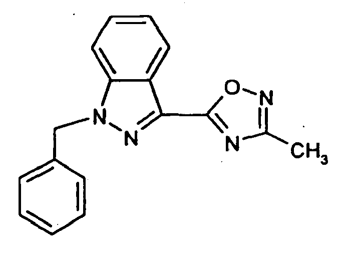 | J3 | 21 % 155°C | 0,29 (H/EE 3:1) |
| III/ 129 | 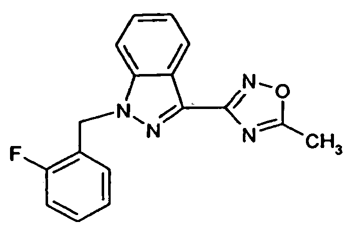 | J3 | | |
| III/ 130 | 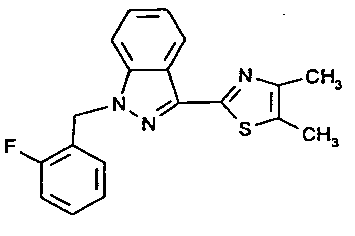 | B3 | 38 % 131°C | 0,46 (Cy:EE 10:1) |
| III/ 131 | 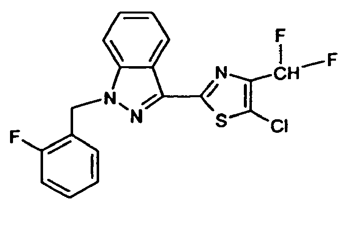 | B3 | 86 % 123°C | 0,43 (Cy:EE 10:1) |
| III/ 132 | 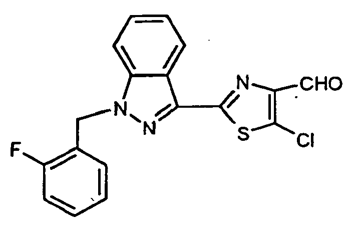 | B3 | 78 % 166°C | 0,41 (Cy:EE 10:1) |
| III/ 133 | 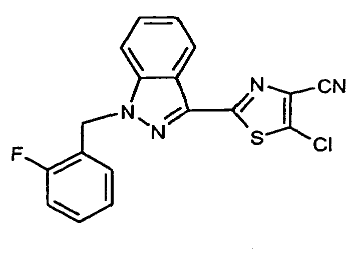 | B3 | 73 % 162°C | 0,43 (Cy:EE 10:1) |

### Beispiele des erfindungsgemäßen Verfahrens zur Herstellung der Oxazolylverbindungen der allgemeinen Formel III-XXIX sowie Verbindungen, in denen R⁴² einen Rest der Formel III-XXVIII darstellt:

In den folgenden experimentellen Beschreibungen sind die Retentionsfaktoren R_{f} auf Kieselgel-DC gemessen, wenn nicht anders angegeben. H = Hexan, EE = Essigester.

### Verfahrensbeispiel 1

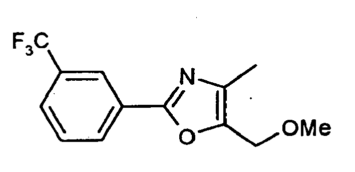

Eine Mischung von 10 g 3-(m-Trifluormethylbenzoylamido)-1,1-dichlor-but-1-en, 5,1 g Natriummethylat und 35 ml Dimethylacetamid wird über Nacht bei 25°C gerührt, dann mit 50 ml Wasser versetzt und mehrfach mit Dichlormethan extrahiert. Die Dichlormethanphase wird abgetrennt, mit Natriumsulfat getrocknet, filtriert und das Lösungsmittel im Vakuumrotationsverdampfer entfernt. Die Destillation des erhaltenen Rohprodukts ergibt 7,3 g 4-Methyl-5-methoxymethyl-2-(m-trifluorphenyl)-oxazol (Siedebereich 96-100°C / 0,2 mbar).

### Herstellung des eingesetzten 1,1-Dichlor-3-(m-trifluormethylbenzovlamido)-but-1-en:

### 1. Stufe

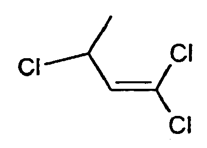

Eine Mischung aus 615 g 1,1,1,3-Tetrachlorbutan, erhalten durch radikalisch initiierte Addition von Tetrachlorkohlenstoff an Propen, 13,3 g Tetrabutylammoniumbromid und einer Lösung von 138,2 g Natriumhydroxid in 390 ml Wasser wird bei Raumtemperatur 24 h durch Rühren gut vermischt. Dann werden 21 Wasser zugegeben, die Phasen getrennt und die organische Phase mit Natriumsulfat getrocknet. Durch Destillation des Rohproduktes erhält man ein Gemisch von 1,1,3-Trichlor-but-1-en und 1,1,1-Trichlor-but-2-en, (492 g vom Siedebereich 45-50°C / 20 mbar).

### 2. Stufe

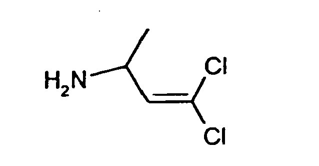

210 g eines Gemisches von 1,1,3-Trichlor-but-1-en und 1,1,1-Trichlor-but-2-en und 52 ml wasserfreie Blausäure werden gleichzeitig innerhalb 1 Stunde zu einer auf 40°C erwärmten Lösung von 38 g H₂O in 568 g konzentrierter Schwefelsäure unter Rühren zudosiert. Dann werden innerhalb von 2 Stunden weitere 78 ml Blausäure hinzugegeben. Nach weiteren 2 Stunden Reaktionszeit wird die überschüssige Blausäure abdestilliert. Mit 20%iger Natronlauge wird das Reaktionsgemisch alkalisch gestellt und das Rohprodukt (195 g) durch Extraktion mit Dichlormethan abgetrennt.

Dieses Rohprodukt wird unter Rühren mit 950 ml halbkonzentrierter Salzsäure vermischt, wobei unter Rückflußkühlung zum Sieden erhitzt wird. Nach 24 Stunden wird abgekühlt, eine geringe Menge an Nebenprodukten durch Extraktion mit Dichlormethan entfernt. Die wäßrige Phase wird im Rotationsverdampfer zur Trockene eingeengt. Dann wird mit halbkonzentrierter Natronlauge versetzt und verrührt, wobei der pH-Wert auf 9 gestellt wird. Das sich abscheidende Amin wird isoliert und destilliert. Man erhält 156 g 3-Amino-1,1-dichlor-but-1-en, Kp 45-50°C / 18 mbar.

### 3. Stufe

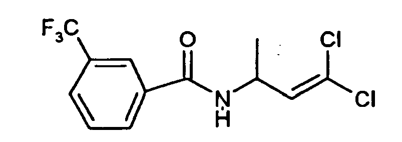

Zu einer Mischung von 21,0 g 3-Amino-1,1-dichlor-but-1-en, 35 ml Dichlormethan und einer Lösung von 15,9 g Natriumcarbonat in 45 ml Wasser wird unter Eiskühlung und kräftigem Rühren eine Lösung von 26,9 g m-Trifluormethylbenzoylchlorid in 25 ml Dichlormethan innerhalb von 30 Minuten zugetropft. Nach einer weiteren Stunde Reaktionszeit werden die Phasen getrennt. Durch Einengen der organischen Phase erhält man 39,0 g 2,2-Dichlor-3-(m-trifluormethylbenzoylamido)-but-1-en.

### Verfahrensbeispiel 2

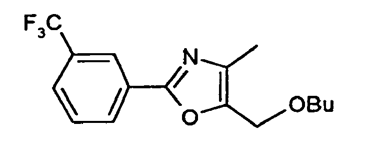

Eine Mischung von 1,1-Dichlor-4-(m-trifluormethylbenzoylamido)-but-1-en, 4,2 g Natriumbutylat und 35 ml Dimethylacetamid wird unter Rühren 7 h auf 100°C erwärmt. Nach der Umsetzung wird analog Beispiel 1 aufgearbeitet. Die Vakuumdestillation des erhaltenen Rohproduktes ergibt 6,4 g 5-Butoxymethyl-4-methyl-2-(m-trifluorphenyl)-oxazol (Siedebereich 106-110°C / 0,2 mbar).

### Verfahrensbeispiel 3

Eine Lösung von 1,8 g 3-Acetamido-1,1-dichlor-but-1-en und 1,0 g Natriummethylat in 20 ml Methanol wird 24 Stunden unter Rückfluß zum Sieden erhitzt. Darauf wird das Methanol abdestilliert, der Rückstand mit 5 ml Wasser und 30 ml Dichlormethan aufgenommen, die organische Phase abgetrennt und im Vakuum destilliert. Man erhält 0,9 g 2,4-Dimethyl-5-methoxymethyl-oxazol, Kp 54°C / 20 mbar.

### Verfahrensbeispiel 4

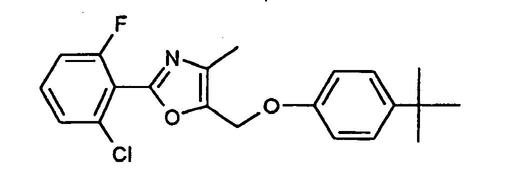

Eine Mischung von 7 g 3-(2-Chlor-6-fluor-benzoylamido)-1,1-dichlor-but-1-en, 11,7 g Natrium-4-tert.butyl-phenolat und 100 ml N-Methylpyrrolidon wird 8 Stunden unter Rühren auf 100°C erwärmt. Anschließend wird das Lösungsmittel durch Vakuumdestillation abgetrennt, der Rückstand mit Wasser und Dichlormethan aufgenommen, die organische Phase abgetrennt, mit Natriumsulfat getrocknet und im Vakuumrotationsverdampfer vom Lösungsmittel befreit. Das Rohprodukt wird chromatographisch gereinigt. Man erhält 5,1 g 2-(2-Chlor-6-fluor-phenyl)-4-methyl-5-(4-tert.butyl-phenoxymethyl)-oxazol.

### Verfahrensbeispiel 5

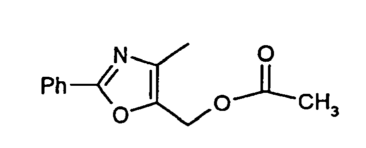

Eine Mischung von 5 g 3-Benzoylamido-1,1-dichlor-but-1-en, 36 g Natriumacetat und 500 ml N-Methylpyrrolidon wird 36 Stunden unter Rühren auf 150°C erwärmt. Anschließend wird analog wie in Beispiel 4 angegeben das Rohprodukt isoliert. Durch Vakuumdestillation erhält man 35 g 5-Acetoxymethyl-4-methyl-2-phenyl-oxazol (Siedebereich 88-91 °C / 0,1 mbar).

### Verfahrensbeispiel 6

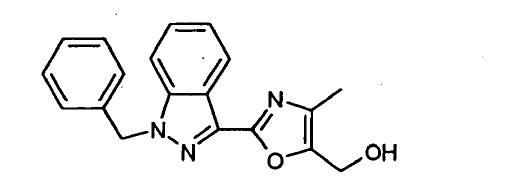

15 mg 3-(1-Benzylindazol-3-carboxamido)-1,1-dichlor-but-1-en (40 µmol) in 20 µl N-Methypyrrolidon unter Argon wurden mit 80 µl NaOH 1M versetzt und die Mischung 1 h auf 55°C erhitzt. Die Mischung wurde abgekühlt und mit 0,8 ml Wasser und 8 ml Essigester versetzt. Die organische Phase wurde eingeengt und der Rückstand durch präparative DC (SiO₂) gereinigt. 9,9 mg (77 %) 2-(1-Benzyl-indazol-3-yl)-5-hydroxymethyl-4-methyl-oxazol wurden erhalten (mp. 127-129°C). MS (DCl/NH₃): 320 (100, MH⁺). R_{f}: 0,17 (H:EE 1:1).

### Verfahrensbeispiel 7

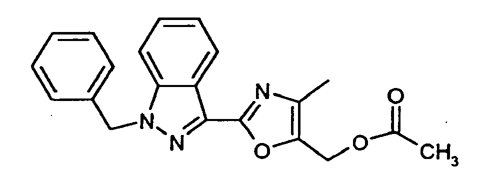

(Für dieses Beispiel wurde die Amid-Zwischenstufe in situ aus der Formel 3a und einem Säurechlorid hergestellt.)

500 mg 1-Benzylindazol-3-carboxylchlorid (1,847 mmol), 260 mg 3-Amino-1,1-dichlor-but-1-en (1,847 mmol) und 318 mg Natriumacetat (3,88 mmol) wurden in 4 ml N-Methylpyrrolidon unter Argon 5 Tage bei 150°C gerührt. Die Rohmischung wurde abgekühlt, mit Wasser versetzt und mehrmals mit Essigester extrahiert. Die organischen Phasen wurden getrocknet (auf Na₂SO₄) und eingeengt, und der Rückstand chromatographiert (SiO₂, Petrolether-Essigester 3:1). Zwei Produkte wurden isoliert: 1,1-Dichlor-3-(1-benzylindazol-3-carboxamido)-but-1-en (410 mg, 59 %) R_{f}: 0,26 (H:EE 3:1) und 5-Acetoxymethyl-2-(1-benzylindazol-3-yl)-4-methyloxazol (160 mg, 24 %).
MS (DCl/NH₃): 362 (100, MH⁺). R_{f}: 0,17 (H:EE 3:1).

### Verfahrensbeispiel 8

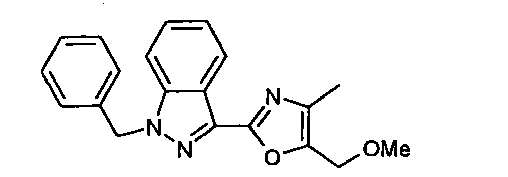

100 mg 3-(1-Benzylindaol-3-carboxamido)-1,1-dichlor-but-1-en (0,267 mmol) und 29 mg Natriummethylat wurden in 0,5 ml N-Methylpyrrolidon unter Argon über Nacht bei 100°C gerührt. Die Rohmischung wurde abgekühlt und direkt durch Säulenchromatographie gereinigt (SiO₂, Cyclohexan:Essigester 3:1). Isoliert wurden 35,9 mg (40 %) 2-(1-Benzylindazol-3-yl)-5-methoxymethyl-4-methyl-oxazol als gelbliches Öl.
MS (DCl/NH₃): 334 (100, MH⁺). R_{f}: 0,67 (CH₂Cl₂:MeOH 100:5).

### Verfahrensbeispiel 9

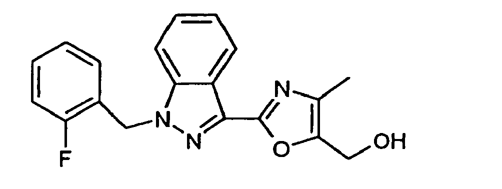

730 mg 1,1-Dichlor-3-[1-(2-fluorbenzyl)indazol-3-carboxamido]-but-1-en (1,86 mmol) und 3,75 ml NaOH 1N (3,75 mmol) wurden in 7,4 ml N-Methylpyrrolidon unter Argen über Nacht bei 50°C gerührt. Nach dem Abkühlen wurde die Mischung auf Eiswasser gegossen und das ausgefallene Produkt abfiltriert und getrocknet. Schließlich wurde es durch Säulenchromatographie (SiO₂, Cyclohexan:Essigester 2:1) gereinigt. Erhalten wurden 375 mg (60 %) 2-[1-(2-Fluorbenzyl)indazol-3-yl]-5-hydroxymethyl4-methyl-oxazol als weiße Kristalle. Mp. 144°C.
MS (ESI-POSITIV): 338 (100, MH⁺). R_{f}: 0,20 (H:EE 1:1).

Das eingesetzte 1,1-Dichlor-3-[1-(2-fluorbenzyl)indazol-3-carboxamido]-but-1-en wurde folgenderweise hergestellt: 400 mg 1-(2-Fluorobenryl)-indazol-3-carboxyl-chlorid (1,385 mmol) und 200 mg 3-Amino-1,1-dichlor-but-1-en in 1,5 ml THF wurden mit 120 µl Pyridin versetzt und 3 h bei RT gerührt. Anschließend wurde mit Essigester und Wasser versetzt. Die organische Phase wurde auf Natriumsulfat getrocknet und eingeengt. Das Rohprodukt 1,1-Dichlor-3-[1-(2-fluorbenzyl)indazol-3-carboxamido]-but-1-en ist laut DC sauber genug, um direkt weiter umgesetzt zu werden.
R_{f}: 0,32 (H:EE 3:1).

### Verfahrensbeispiel 10

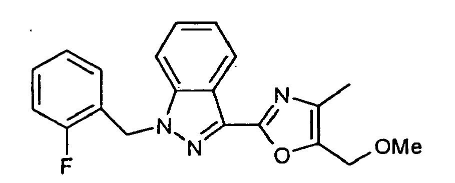

136 mg 1,1-Dichlor-3-[1-(2-fluorbenzyl)indazol-3-carboxamido]-but-1-en (0,267 mmol) und 47 mg Natriummethylat wurden in 0,6 ml N-Methylpyrrolidon unter Argon über Nacht bei 100°C gerührt. Die Rohmischung wurde abgekühlt und direkt durch Säulenchromatographie gereinigt (SiO₂). Isoliert wurden 24 mg (20 %) 2-[1-(2-Fluorobenzyl)-indazol-3-yl]-5-methoxymethyl-4-methyl-oxazol als gelbliche Kristalle.
Mp. 86-88°C. MS (ESI-POSITIV): 374 (65, M+Na⁺), 352 (100, MH⁺). R_{f}: 0,18 (CH₂Cl₂:MeOH 100:1).

### Verfahrensbeispiel 11

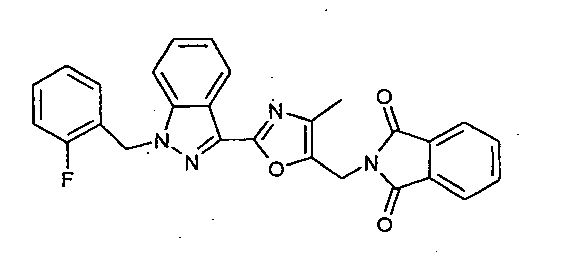

136 mg 1,1-Dichlor-3-[1-(2-fluorbenzyl)indazol-3-carboxamido]-but-1-en (0,267 mmol) und 164 mg Kaliumphthalimid wurden in 0,6 ml N-Methylpyrrolidon unter Argon über Nacht bei 150°C gerührt. Anschließend wurde das N-Methylpyrrolidon im Hochvakuum bei 60°C abgezogen und der Rückstand chromatographiert (SiO₂, Cyclohexan/Essigester 2:1), um 48,5mg (36 %) 2-[1-(2-Fluorobenzyl)-indazol-3-yl]-4-methyl-5-(N-phthalimidomethyl)-oxazol zu erhalten. Gelbliche Kristalle.
Mp. 175-177°C. MS (ESI-POSITIV): 467 (100, MH⁺). R_{f}: 0,64 (CH₂Cl₂:MeOH 100:1).

### Verfahrensbeispiel 12

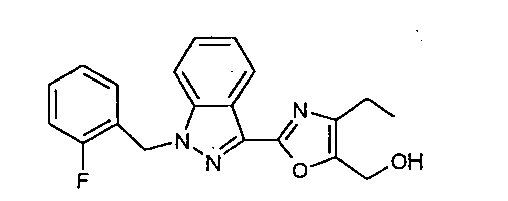

1,05 g 1,1-Dichlor-3-[1-(2-fluorbenzyl)indazol-3-carboxamido]-pent-1-en (2,58 mmol) und 5,20 ml NaOH 1N (5,20 mmol) wurden in 15 ml N-Methylpyrrolidon unter Argon 2 Tage bei 50°C gerührt. Nach dem Abkühlen wurde die Mischung auf Eiswasser gegossen und die Mischung mehrmals mit Essigester extrahiert. Die organische Phase wurde getrocknet (Natriumsulfat) und eingeengt (N-Methylpyrrolidon im Hochvakuum abgezogen). Der Rückstand wurde durch Säulenchromatographie (Aluminiumoxid, Cyclohexan/Essigester 2:1) gereinigt. Erhalten wurden 470 mg (52 %) 5-Ethyl-2-[1-(2-fluorobenzyl)-indazol-3-yl]-5-hydroxymethyl-oxazol als weiße Kristalle.
Mp. 137-139°C. MS (ESI-POSITIV): 352 (100, MH⁺). R_{f}: 0,08 (Aluminiumoxid, Cyclohexan:EE 2:1).

## Patentansprüche

1. 3-Heterocyclyl-substituierte Pyrazolderivate der allgemeinen Formel (III-I) 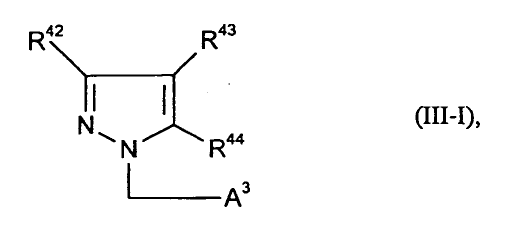 in welcher
R⁴² für Imidazolyl, Oxazolyl, Thiazolyl oder Oxadiazolyl steht, die gegebenenfalls bis zu 2-fach gleich oder verschieden durch Ethoxycarbonyl, Phenyl oder durch Methyl oder Ethyl substituiert sind, wobei die Alkylreste ihrerseits durch Hydroxy, Chlor, Ethoxycarbonyl, Oxycarbonylmethyl oder Methoxy substituiert sein können,
R⁴³ und R⁴⁴ gemeinsam unter Einbezug der Doppelbindung für Phenyl stehen, das gegebenenfalls durch Nitro substituiert ist,
A³ für Phenyl oder Fluor substituiertes Phenyl oder Pyrimidyl steht,
und deren Isomere und Salze.

2. Verfahren zur Herstellung der Verbindungen nach Anspruch 1 der allgemeinen Formel (III-I), **dadurch gekennzeichnet, daß** man
[A3] Verbindungen der allgemeinen Formel (III-II) 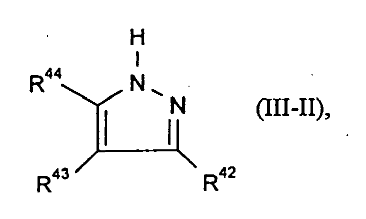 in welcher
R⁴², R⁴³ und R⁴⁴ die oben angegebene Bedeutung haben,
mit Verbindungen der allgemeinen Formel (III-III)
D³-CH₂-A³ (III-III),
in welcher
A³ die oben angegebene Bedeutung hat,
und
D³ für Triflat oder Halogen, vorzugsweise für Brom steht,
in inerten Lösemitteln, gegebenenfalls in Anwesenheit einer Base umsetzt,
oder
[B3] Verbindungen der allgemeinen Formel (III-IV) 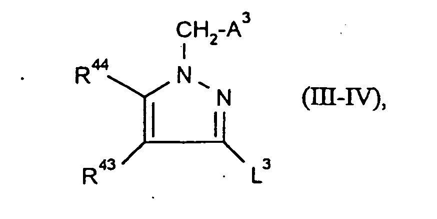 in welcher
A³, R⁴³ und R⁴⁴ die oben angegebene Bedeutung haben,
und
L³ für einen Rest der Formel -SnR⁵⁵R⁵⁶R⁵⁷, ZnR⁵⁸, Iod, Brom oder Triflat steht,
worin
R⁵⁵, R⁵⁶ und R⁵⁷ gleich oder verschieden sind und geradkettiges oder verzweigtes Alkyl mit bis zu 4 Kohlenstoffatomen bedeuten,
und
R⁵⁸ Halogen bedeutet,
mit Verbindungen der allgemeinen Formel (III-V)
R⁴²-T³ (III-V),
in welcher
R⁴² die oben angegebene Bedeutung hat
und
im Fall L³ = SnR⁵⁵R⁵⁶R⁵⁷ oder ZnR⁵⁸
T³ für Triflat oder für Halogen, vorzugsweise für Brom steht,
und
im Fall L³ = Jod, Brom oder Triflat
T³ für einen Rest der Formel SnR^{55'}R^{56'}R^{57'}, ZnR^{58'} oder BR⁵⁹R⁶⁰ steht,
worin
R^{55'}, R^{56'}, R^{57'} und R^{58'} die oben angebene Bedeutung von R⁵⁵, R⁵⁶, R⁵⁷ und R⁵⁸ haben und mit dieser gleich oder verschieden sind,
R⁵⁹ und R⁶⁰ gleich oder verschieden sind und Hydroxy, Aryloxy mit 6 bis 10 Kohlenstoffatomen oder geradkettiges oder verzweigtes Alkyl oder Alkoxy mit jeweils bis zu 5 Kohlen stoffatomen bedeuten, oder gemeinsam einen 5- oder 6-gliedrigen carbocyclischen Ring bilden,
in einer palladiumkatalysierten Reaktion in inerten Lösemitteln umsetzt,
oder
[C3] im Fall R⁴² = 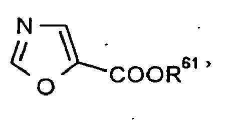 in welchen
R⁶¹ für geradkettiges oder verzweigtes Alkyl mit bis zu 4 Kohlenstoffatomen steht,
Verbindungen der allgemeinen Formel (III-VI) 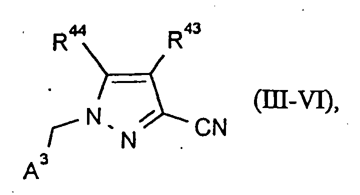 in welcher
A³, R⁴³ und R⁴⁴ die oben angegebene Bedeutung haben,
mit Diazoverbindungen der allgemeinen Formel (III-VII) 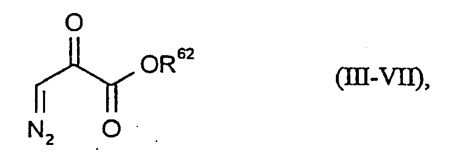 in welcher
R⁶² für geradkettiges oder verzweigtes Alkyl mit bis zur 4 Kohlenstoffatomen steht,
in Gegenwart von Kupfersalzen oder Rhodiumsalzen zu Verbindungen der allgemeinen Formel (III-Ia) 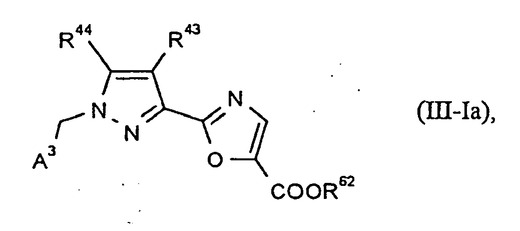 in welcher
A³, R⁴³, R⁴⁴ und R⁶² die oben angegebene Bedeutung haben,
umsetzt,
[D3] im Fall R⁴²'= 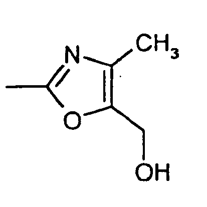 Verbindungen der allgemeinen Formel (III-VIII) 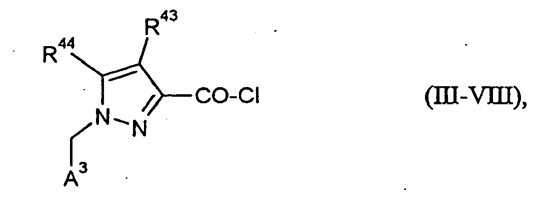 in welcher
A³, R⁴³ und R⁴⁴ die oben angegebene Bedeutung haben,
entweder direkt durch Umsetzung mit der Verbindung der Formel (III-IX) 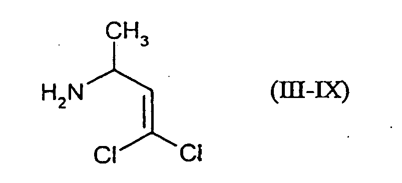 in dem System NaOCO-CH₃/N-Methylpyrrolidin
in die Verbindungen der allgemeinen Formel (III-Ib) 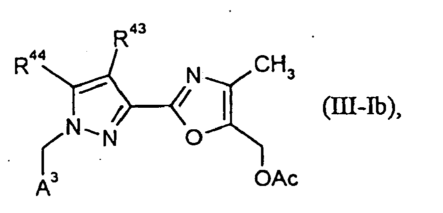 in welcher
R⁴³, R⁴⁴ und A³ die oben angegebene Bedeutung haben,
überführt,
und anschließend durch Einwirkung von Kaliumhydroxid in Methanol die Acetylgruppe abspaltet,
oder
zunächst durch Umsetzung der Verbindungen der allgemeinen Formel (III-VIII) mit der Verbindung der Formel (III-IX) die Verbindungen der allgemeinen Formel (III-X) 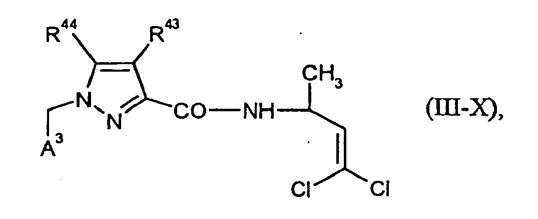 in welcher
R⁴³, R⁴⁴ und A³ die oben angegebene Bedeutung haben,
herstellt,
und in einem weiteren Schritt durch Einwirkung von Kaliumhydroxid die Hydroxymethylverbindungen herstellt,
und gegebenenfalls durch eine Alkylierung nach üblichen Methoden in die entsprechende Alkoxyverbindungen überführt,
oder
[E3] Verbindungen der allgemeinen Formel (III-VII) 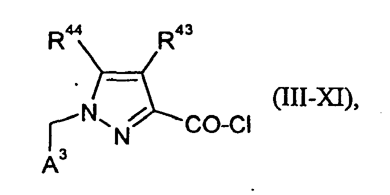 in welcher
A³, R⁴³ und R⁴⁴ die oben angegebene Bedeutung haben,
durch Umsetzung mit der Verbindung der Formel (III-XII) 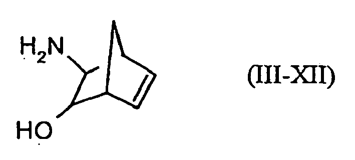 die Verbindungen der allgemeinen Formel (III-XIII) 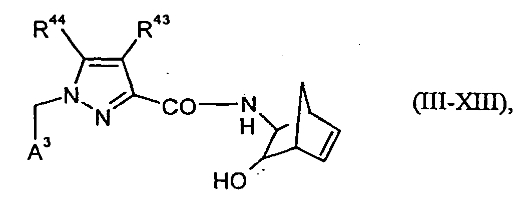 in welcher
A³, R⁴³ und R⁴⁴ die oben angegebene Bedeutung haben,
herstellt,
und anschließend im Sinne einer Retro-Diels-Alderreaktion umsetzt
oder
[F3] Verbindungen der allgemeinen Formel (III-XIV) 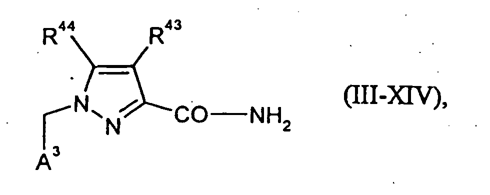 in welcher
A³, R⁴³ und R⁴⁴ die oben angegebene Bedeutung haben,
mit Verbindungen der allgemeinen Formel (III-XV)
Br-CH₂-CO-R⁶³ (III-XV),
in welcher
R⁶³ geradkettiges oder verzweigtes Alkyl oder Alkoxycarbonyl mit jeweils bis zu 4 Kohlenstoffatomen bedeutet,
in inerten Lösemitteln zu den Verbindungen der allgemeinen Formel (III-Ic) 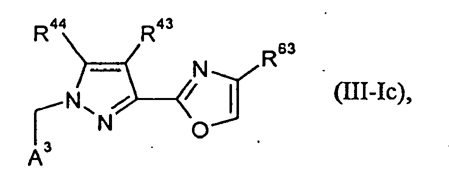 in welcher
A³, R⁴³, R⁴⁴ und R⁶³ die oben angegebene Bedeutung haben,
umsetzt,
und im Fall der Ester (R⁶³ = CO₂-(C₁-C₄-Alkyl), eine Reduktion nach üblichen Methoden zu den entsprechenden Hydroxymethylverbindungen durchführt,
oder
[G3] im Fall R⁴² = 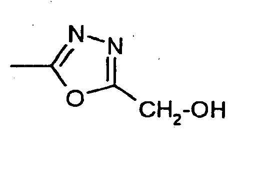 Carbonsäuren der allgemeinen Formel (III-XVI) 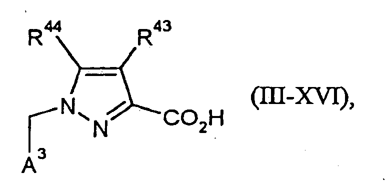 in welcher
A³, R⁴³ und R⁴⁴ die oben angegebene Bedeutung haben,
mit Hydrazinhydrat zunächst in die Verbindungen der allgemeinen Formel (III-XVII) 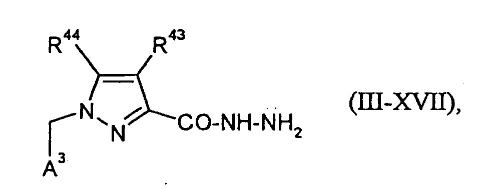 in welcher
A³, R⁴³ und R⁴⁴ die oben angegebene Bedeutung haben,
überführt,
in einem weiteren Schritt mit der Verbindung der Formel (III-XIII)
Cl-CO-CH₂-Cl (III-XVIII)
die Verbindungen der allgemeinen Formel (III-XIX) 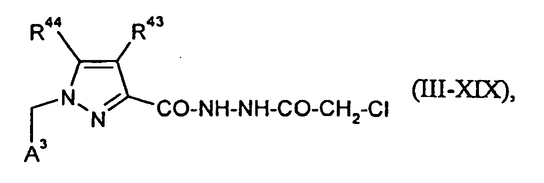 in welcher
A³, R⁴³ und R⁴⁴ die oben angegebene Bedeutung haben,
herstellt,
anschließend unter Einwirkung von Phosphoroxytrichlorid eine Cyclisierung zu den Verbindungen der allgemeinen Formel (III-Id) 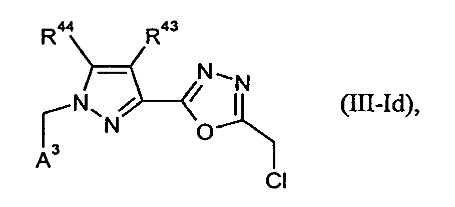 in welcher
A³, R⁴³ und R⁴⁴ die oben angegebene Bedeutung haben,
durchführt,
und wie oben bereits beschrieben über die Stufe der entsprechenden -CH₂-O-CO-CH₃ substituierten Verbindungen die -CH₂-OH substituierten Verbindungen herstellt,
oder
[H3] im Fall, daß R⁴² für einen Rest der Formel 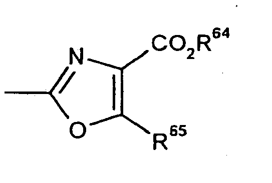 steht,
worin
R⁶⁴ Wasserstoff oder geradkettiges oder verzweigtes Alkyl mit bis zu 4 Kohlenstoffatomen bedeutet und
R⁶⁵ den Bedeutungsumfang der oben unter dem heterocyclischen Rest R⁴² aufgeführten Untersubstituenten umfaßt Verbindungen der allgemeinen Formel (III-XX) 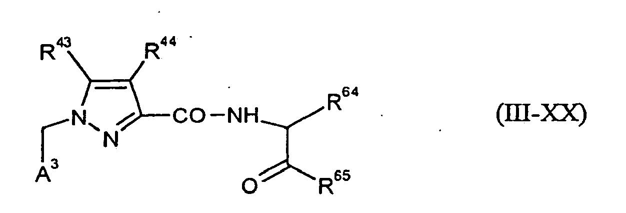
in welcher
A³, R⁴³, R⁴⁴, R⁶⁴ und R⁶⁵ die oben angegebene Bedeutung haben
im System PPh₃/J₂ in Anwesenheit einer Base, vorzugsweise mit Triethylamin umsetzt
oder
[I3] im Fall, daß R⁴² für die Gruppe der Formel 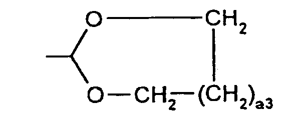 steht, worin a3 die oben angegebene Bedeutung hat
Verbindungen der allgemeinen Formel (III-XXI) 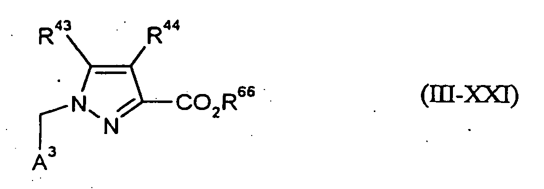 in welcher
A³, R⁴³ und R⁴⁴ die oben angegebene Bedeutung haben und
R⁶⁶ die oben angegebene Bedeutung von R⁶⁴ hat und mit dieser gleich oder verschieden ist,
entweder zunächst durch Reduktion nach üblichen Methoden in die Verbindungen der allgemeinen Formel (III-XXII) 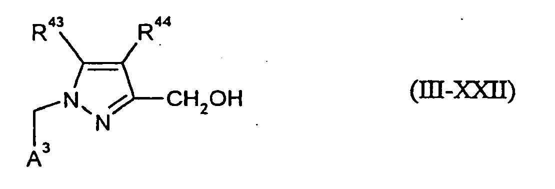 in welcher
A³, R⁴³ und R⁴⁴ die oben angegebene Bedeutung haben,
überführt und anschließend durch Oxidation die Verbindungen der allgemeinen Formel (III-XXIII) 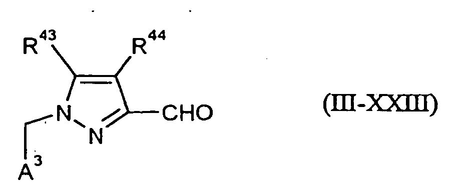 in welcher
A³, R⁴³ und R⁴⁴ die oben angegebene Bedeutung haben,
herstellt oder
direkt die Verbindungen der allgemeinen Formel (III-XXI) durch Reduktion in die Verbindungen der allgemeinen Formel (III-XXIII) überführt
und abschließend mit 1,2- oder 1,3-Dihydroxyverbindungen nach klassischen Methoden umsetzt
oder
[J3] im Fall, daß R⁴² für den Rest der Formel 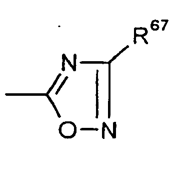 steht,
worin
R⁶⁷ die oben angegebene Bedeutung von R⁶⁵ und mit dieser gleich oder verschieden ist,
entweder Verbindungen der allgemeinen Formel (III-XXIV) 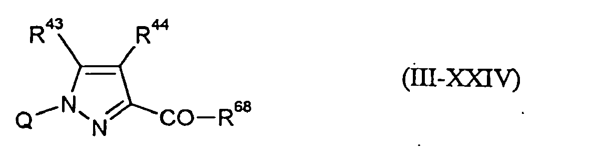 in welcher
R⁴³ und R⁴⁴ die oben angegebene Bedeutung haben und
Q für Wasserstoff oder für den -CH₂-A³-Rest steht und
R⁶⁸ für Halogen oder geradkettiges oder verzweigtes Alkoxy mit bis zu 4 Kohlenstoffatomen, vorzugsweise für Chlor, Methoxy oder Ethoxy steht
mit Verbindungen der allgemeinen Formel (III-XXV) 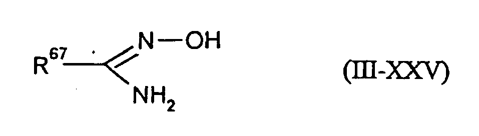 in welcher
R⁶⁷ die oben angegebene Bedeutung hat
gegebenenfalls in Anwesenheit einer Base umsetzt und im Fall Q = H anschließend mit Verbindungen der allgemeinen Formel A³-CH₂-Br (III-XXVI), in welcher A die oben angegebene Bedeutung hat umsetzt oder
Verbindungen der allgemeinen Formel (III-XXVII) 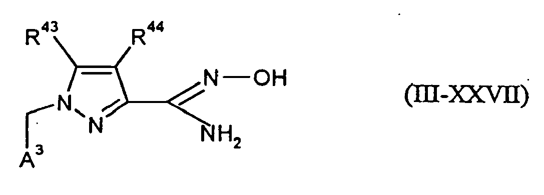 in welcher
A³, R⁴³ und R⁴⁴ die oben angegebene Bedeutung haben
mit Verbindungen der allgemeinen Formel (III-XXVIII)
R^{67'}-CO-R^{68'} (III-XXVIII)
in welcher
R^{67'} die oben angegebene Bedeutung von R⁶⁷ hat und mit dieser gleich oder verschieden ist
und
R^{68'} die oben angegebene Bedeutung von R⁶⁸ hat und mit dieser gleich oder verschieden ist gegebenenfalls in Anwesenheit einer Base umsetzt.
und im Fall der Reste -S(O)_{c3}NR⁵⁰R⁵¹ und -S(O)_{c3'}NR^{50'}R^{51'} ausgehend von den unsubstituierten Verbindungen der allgemeinen Formel (III-I) zunächst eine Umsetzung mit Thionylchlorid und abschließend mit der Aminkomponente durchführt,
und gegebenenfalls die unter R⁴², R⁴³, R⁴⁴ und/oder A³ aufgeführten Substituenten nach üblichen Methoden, vorzugsweise durch Reduktion, Oxidation, Abspaltung von Schutzgruppen und/oder nucleophiler Substitution variiert oder einführt.

3. Arzneimittel enthaltend mindestens eine Verbindung der allgemeinen Formel (III-I) gemäß Anspruch 1.

4. Arzneizubereitungen enthaltend eine Kombination aus mindestens einer Verbindung aus der allgemeinen Formel (III-I) gemäß Anspruch 1 und mindestens einem organischen Nitrat oder einem NO-Donator.

5. Arzneizubereitungen enthaltend eine Kombination aus mindestens einer Verbindung der allgemeinen Formel (III-I) gemäß Anspruch 1 und Verbindungen die den Abbau von cyclischem Guanosinmonophosphat (cGMP) inhibieren.

6. Verwendung von Verbindungen der allgemeinen Formel (III-I) gemäß Anspruch 1 bei der Herstellung von Arzneimitteln zur Behandlung von kardiovaskulären Erkrankungen.

7. Verwendung von Verbindungen der allgemeinen Formel (III-I) gemäß Anspruch 1 bei der Herstellung von Arzneimitteln zur Prophylaxe und Bekämpfung der Folgen cerebraler Infarktgeschehen (Apoplexia cerebri) wie Schlaganfall, cerebraler Ischämien und des Schädel-Hirn-Traumas.

## Claims

1. 3-Heterocyclyl-substituted pyrazole derivatives of the general formula (III-I) 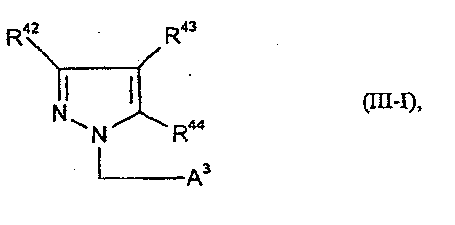 in which
R⁴² represents imidazolyl, oxazolyl, thiazolyl or oxadiazolyl, which are optionally substituted up to twice in an identical or different manner by ethoxycarbonyl, phenyl or by methyl or ethyl, wherein the alkyl radicals in their turn can be substituted by hydroxyl, chlorine, ethoxycarbonyl, oxycarbonylmethyl or methoxy,
R⁴³ and R⁴⁴ together, including the double bond, represent phenyl, which is optionally substituted by nitro,
A³ represents phenyl or fluorine-substituted phenyl or pyrimidyl,
and their isomers and salts.

2. Process for the preparation of the compounds according to Claim 1 of the general formula (III-I), **characterized in that**
[A3]compounds of the general formula (III-II) 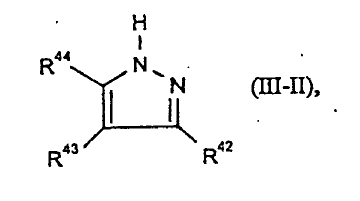 in which
R⁴², R⁴³ and R⁴⁴ have the abovementioned meaning,
are reacted with compounds of the general formula (III-III)
D³-CH₂-A³ (III-III)
in which
A³ has the abovementioned meaning
and
D³ represents triflate or halogen, preferably bromine,
in inert solvents, if appropriate in the presence of a base,
or
[B3]compounds of the general formula (III-IV) 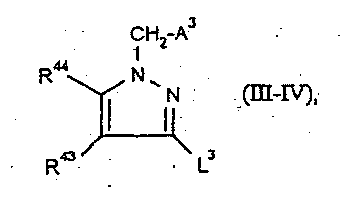 in which
A³, R⁴³ and R⁴⁴ have the abovementioned meaning
and
L³ represents a radical of the formula -SnR⁵⁵R⁵⁶R⁵⁷, ZnR⁵⁸, iodine, bromine or triflate,
wherein
R⁵⁵, R⁵⁶ and R⁵⁷ are identical or different and denote straight-chain or branched alkyl having up to 4 carbon atoms
and
R⁵⁸ denotes halogen,
are reacted with compounds of the general formula (III-V)
R⁴²-T³ (III-V),
in which
R⁴² has the abovementioned meaning
and
in the case where L³ = SnR⁵⁵R⁵⁶R⁵⁷ or ZnR⁵⁸,
T³ represents triflate or represents halogen, preferably bromine,
and
in the case where L³ = iodine, bromine or triflate,
T³ represents a radical of the formula SnR^{55'}R^{56'}R^{57'}, ZnR^{58'} or BR⁵⁹R⁶⁰,
wherein
R⁵⁵', R^{56'}, R^{57'} and R^{58'} have the abovementioned meaning of R⁵⁵, R⁵⁶, R⁵⁷ and R⁵⁸ and are identical to or different from these,
and
R⁵⁹ and R⁶⁰ are identical or different and denote hydroxyl, aryloxy having 6 to 10 carbon atoms or straight-chain or branched alkyl or alkoxy having in each case up to 5 carbon atoms, or together form a 5- or 6-membered carbocyclic ring,
in a palladium-catalysed reaction in inert solvents,
or
[C3] in the case where R⁴² = 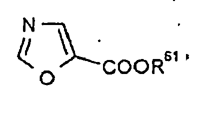 in which
R⁶¹ represents straight-chain or branched alkyl having up to 4 carbon atoms,
compounds of the general formula (III-VI) 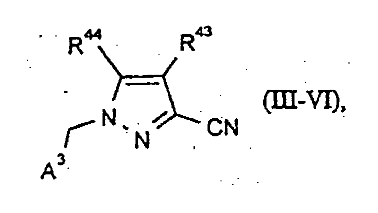 in which
A³, R⁴³ and R⁴⁴ have the abovementioned meaning,
are reacted with diazo compounds of the general formula (III-VII) 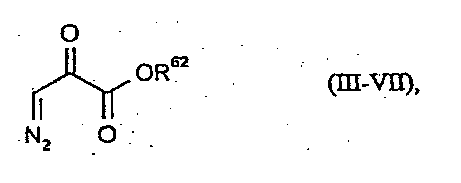 in which
R⁶² represents straight-chain or branched alkyl having up to 4 carbon atoms,
in the presence of copper salts or rhodium salts to give compounds of the general formula (III-Ia) 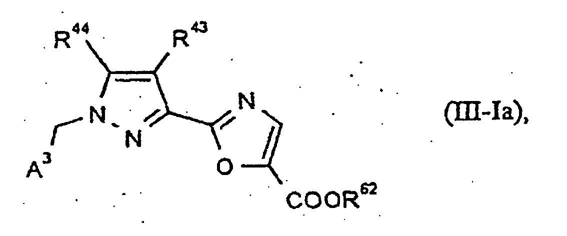 in which
A³, R⁴³, R⁴⁴ and R⁶² have the abovementioned meaning,
[D3] in the case where R⁴² = 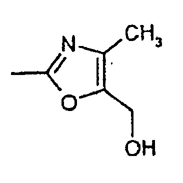 compounds of the general formula (III-VIII) 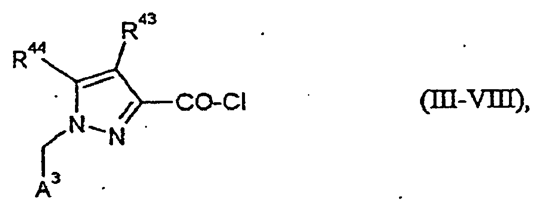 in which
A³, R⁴³ and R⁴⁴ have the abovementioned meaning,
are either converted directly by reaction with the compound of the formula (III-IX) 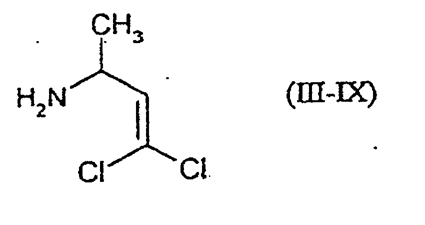 in the system NaOCO-CH₃/N-methylpyrrolidine
into the compounds of the general formula (III-Ib) 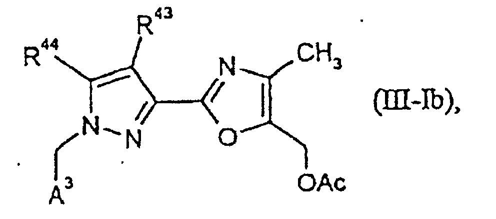 in which
R⁴³, R⁴⁴ and A³ have the abovementioned meaning,
and the acetyl group is then split off by the action of potassium hydroxide in methanol,
or
by reaction of the compounds of the general formula (III-VIII) with the compound of the formula (III-IX), the compounds of the general formula (III-X) 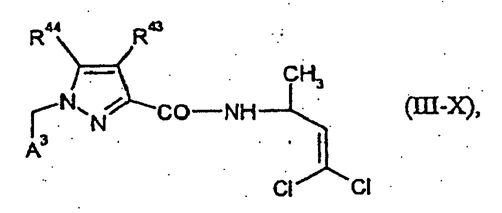 in which
R⁴³, R⁴⁴ and A³ have the abovementioned meaning,
are first prepared,
and the hydroxymethyl compounds are prepared in a further step by the action of potassium hydroxide,
and, if appropriate, are converted into the corresponding alkoxy compounds by an alkylation by customary methods,
or
[E3]compounds of the general formula (III-VII) 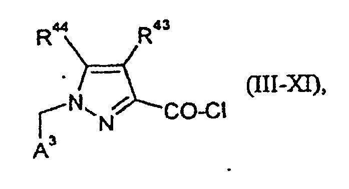 in which
A³, R⁴³ and R⁴⁴ have the abovementioned meaning,
by reaction with the compound of the formula (III-XII) 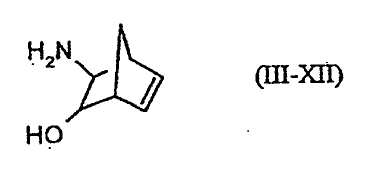 the compounds of the general formula (III-XIII) 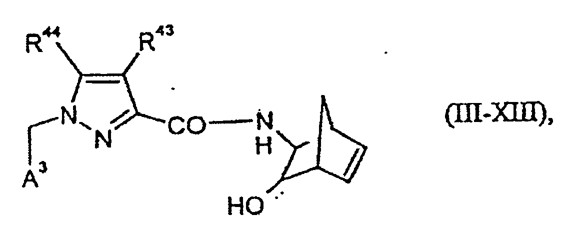 in which
A³, R⁴³ and R⁴⁴ have the abovementioned meaning,
are prepared,
and are then reacted in the context of a retro-Diels-Alder reaction,
or
[F3] compounds of the general formula (III-XIV) 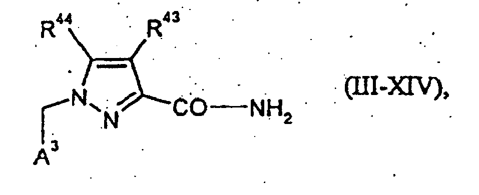 in which
A³, R⁴³ and R⁴⁴ have the abovementioned meaning,
are reacted with compounds of the general formula (III-XV)
Br-CH₂-CO-R⁶³ (III-XV),
in which
R⁶³ denotes straight-chain or branched alkyl or alkoxycarbonyl having in each case up to 4 carbon atoms,
in inert solvents to give the compounds of the general formula (III-Ic) 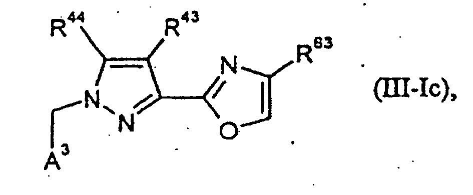 in which
A³, R⁴³, R⁴⁴ and R⁶³ have the abovementioned meaning,
and, in the case of the esters (R⁶³ = CO₂-(C₁-C₄-alkyl), a reduction is carried out by customary methods to give the corresponding hydroxymethyl compounds,
or
[G3]in the case where R⁴² = 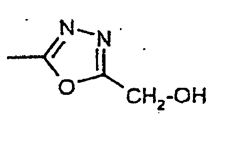 carboxylic acids of the general formula (III-XVI) 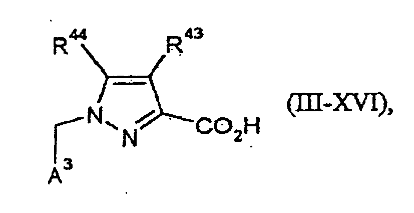 in which
A³, R⁴³ and R⁴⁴ have the abovementioned meaning,
are first converted with hydrazine hydrate into the compounds of the general formula (III-XVII) 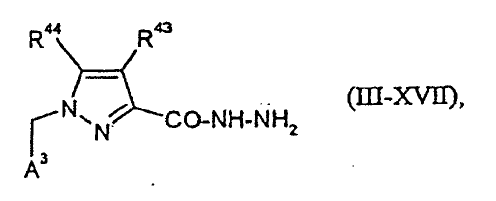 in which
A³, R⁴³ and R⁴⁴ have the abovementioned meaning,
in a further step, with the compound of the formula (III-XVIII)
Cl-CO-CH₂-Cl (III-XVIII)
the compounds of the general formula (III-XIX) 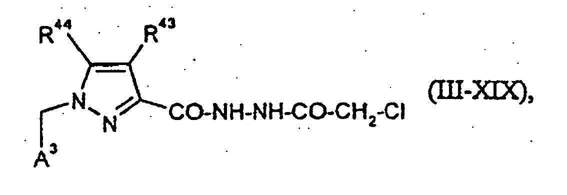 in which
A³, R⁴³ and R⁴⁴ have the abovementioned meaning,
are prepared,
then, under the action of phosphorus oxytrichloride, cyclization is carried out to give the compounds of the general formula (III-Id) 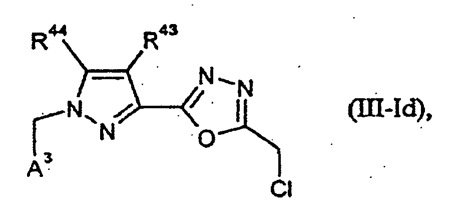 in which
A³, R⁴³ and R⁴⁴ have the abovementioned meaning,
and, as already described above, the -CH₂-OH-substituted compounds are prepared via the stage of the corresponding -CH₂-O-CO-CH₃-substituted compounds,
or
[H3] in the case where R⁴² represents a radical of the formula 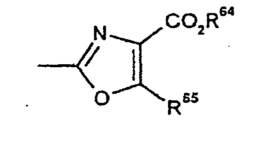 wherein
R⁶⁴ denotes hydrogen or straight-chain or branched alkyl having up to 4 carbon atoms and
R⁶⁵ has the scope of meaning of the secondary substituents listed above under the heterocyclic radical R⁴²,
compounds of the general formula (III-XX) 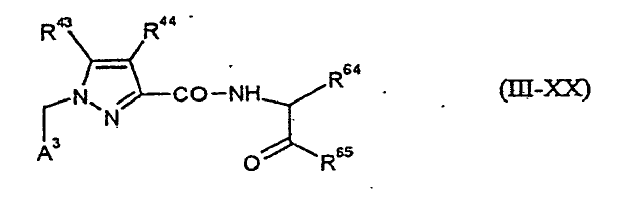 in which
A³, R⁴³, R⁴⁴, R⁶⁴ and R⁶⁵ have the abovementioned meaning,
are reacted in the system PPh₃/I₂ in the presence of a base, preferably with triethylamine,
or
[I3] in the case where R⁴² represents the group of the formula 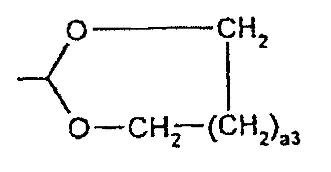 wherein a3 has the abovementioned meaning,
compounds of the general formula (III-XXI) 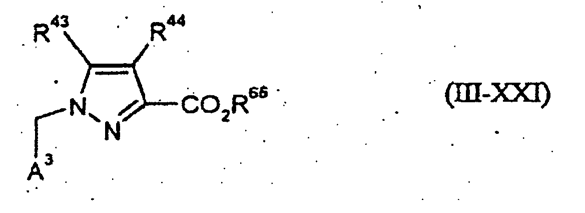 in which
A³, R⁴³ and R⁴⁴ have the abovementioned meaning and
R⁶⁶ has the abovementioned meaning of R⁶⁴ and is identical to or different from this,
either are first converted by reduction by customary methods into the compounds of the general formula (III-XXII) 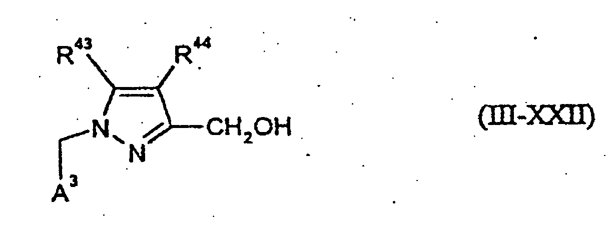 in which
A³, R⁴³ and R⁴⁴ have the abovementioned meaning,
and the compounds of the general formula (III-XXIII) 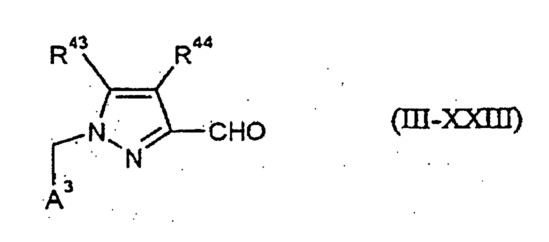 in which
A³, R⁴³ and R⁴⁴ have the abovementioned meaning,
are then prepared by oxidation, or
the compounds of the general formula (III-XXI) are converted directly by reduction into the compounds of the general formula (III-XXIII),
and, finally, these are reacted with 1,2- or 1,3-dihydroxy compounds by conventional methods,
or
[J3] in the case where R⁴² represents the radical of the formula 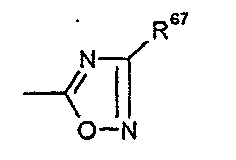 wherein
R⁶⁷ has the abovementioned meaning of R⁶⁵ and is identical to or different from this,
either compounds of the general formula (III-XXIV) 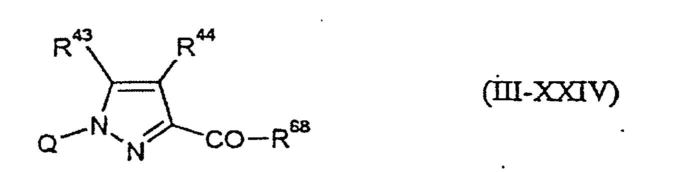 in which
R⁴³ and R⁴⁴ have the abovementioned meaning and
Q represents hydrogen or represents the -CH₂-A³ radical and
R⁶⁸ represents halogen or straight-chain or branched alkoxy having up to 4 carbon atoms, preferably chlorine, methoxy or ethoxy,
are reacted with compounds of the general formula (III-XXV) 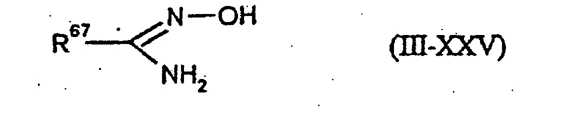 in which
R⁶⁷ has the abovementioned meaning,
if appropriate in the presence of a base, and, in the case where Q = H, the products are then reacted with compounds of the general formula A³-CH₂-Br (III-XXVI), in which A has the abovementioned meaning, or
compounds of the general formula (III-XXVII) 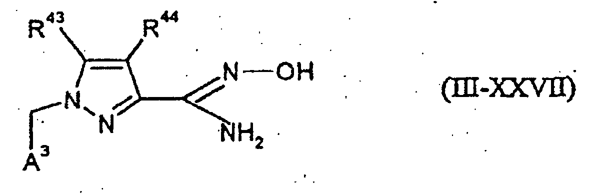 in which
A³, R⁴³ and R⁴⁴ have the abovementioned meaning
are reacted with compounds of the general formula (III-XXVIII)
R^{67'}-CO-R^{68'} (III-XXVIII)
in which
R^{67'} has the abovementioned meaning of R⁶⁷ and is identical to or different from this
and
R^{68'} has the abovementioned meaning of R⁶⁸ and is identical to or different from this, if appropriate in the presence of a base,
and, in the case of the radicals -S(O)_{c3}NR⁵⁰R⁵¹ and - S(O)_{c3'}NR^{50'}R^{51'} starting from the unsubstituted compounds of the general formula (III-I), a reaction first with thionyl chloride and finally with the amine component is carried out,
and, if appropriate, the substituents listed under R⁴², R⁴³, R⁴⁴ and/or A³ are varied or introduced by customary methods, preferably by reduction, oxidation, splitting off of protective groups and/or nucleophilic substitution.

3. Medicaments comprising at least one compound of the general formula (III-I) according to Claim 1.

4. Medicament formulations comprising a combination of at least one compound of the general formula (III-I) according to Claim 1 and at least one organic nitrate or an NO donor.

5. Medicament formulations comprising a combination of at least one compound of the general formula (III-I) according to Claim 1 and compounds which inhibit the breakdown of cyclic guanosine monophosphate (cGMP).

6. Use of compounds of the general formula (III-I) according to Claim 1 in the preparation of medicaments for treatment of cardiovascular diseases.

7. Use of compounds of the general formula (III-I) according to Claim 1 in the preparation of medicaments for prophylaxis and combating the consequences of cerebral infarction events (apoplexia cerebri), such as apoplexy, cerebral ischaemias and cranio-cerebral trauma.

## Revendications

1. Dérivés de pyrazole à substituant 3-hétérocyclyle, de formule générale (III-I) 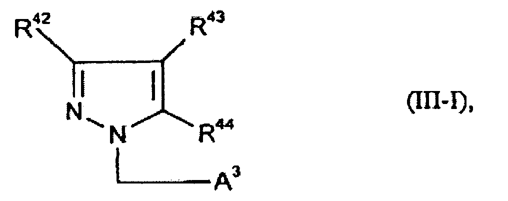 dans laquelle
R⁴² représente des groupes imidazolyle, oxazolyle, thiazolyle
ou oxadiazolyle, qui sont éventuellement substitués jusqu'à deux fois identiques ou différentes par un reste éthoxycarbonyle, phényle ou par un reste méthyle
ou éthyle, les restes alkyle pouvant eux-mêmes être substitués par un radical hydroxy, chloro, éthoxycarbonyle, oxycarbonylméthyle ou méthoxy,
R⁴³ et R⁴⁴ forment ensemble, y compris la double liaison, un groupe phényle qui est éventuellement substitué par un radical nitro,
A³ représente un reste phényle ou pyrimidyle substitué par un radical phényle ou fluoro,
et leurs isomères et leurs sels.

2. Procédé de production des composés suivant la revendication 1, de formule générale (III-I), **caractérisé en ce que**
[A3] des composés de formule générale (III-II) 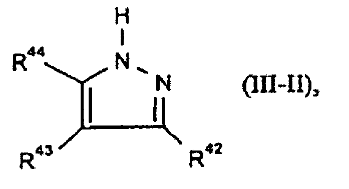 dans laquelle
R⁴², R⁴³ et R⁴⁴ ont la définition indiquée ci-dessus,
sont amenés à réagir avec des composés de formule générale (III-III)
D³-CH₂-A³ (III-III)
dans laquelle
A a la définition indiquée ci-dessus
et
D³ est un groupe triflate ou un halogène, avantageusement le brome,
dans des solvants inertes, avantageusement en présence d'une base,
ou bien
[B3] des composés de formule générale (III-IV) 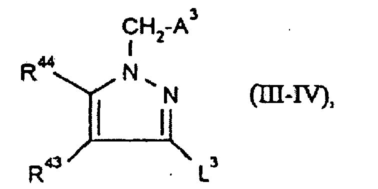 dans laquelle
A³, R⁴³ et R⁴⁴ ont la définition indiquée ci-dessus
et
L³ représente un reste de formule -SnR⁵⁵R⁵⁶R⁵⁷, ZnR⁵⁸, l'iode, le brome ou un groupe triflate,
où
R⁵⁵, R⁵⁶ et R⁵⁷ sont identiques ou différents et représentent un reste alkyle linéaire ou ramifié ayant jusqu'à 4 atomes de carbone
et
R⁵⁸ représente un halogène, sont amenés à réagir avec des composés de formule générale (III-V)
R⁴²-T³ (III-V)
dans laquelle
R⁴² a la définition indiquée ci-dessus
et
dans le cas L³ = SnR⁵⁵R⁵⁶R⁵⁷ ou ZnR⁵⁸,
T³ représente un groupe triflate ou un halogène
et
dans le cas L³ = iode, brome ou groupe triflate,
T³ représente un reste de formule SnR^{55'}R^{56'}R^{57'} ZnR^{58'} ou BR⁵⁹R⁶⁰,
où
R^{55'}, R^{56'}, R^{57'}et R^{R8'} ont la définition donnée ci-dessus pour R⁵⁵, R^{56'}, R⁵⁷ et R⁵⁸ et y sont identiques ou en sont différents,
R⁵⁹ et R⁶⁰ sont identiques ou différents et représentent un reste hydroxy, aryloxy ayant 6 à 10 atomes de carbone ou un reste alkyle ou alkoxy linéaire ou ramifié ayant chacun jusqu'à 5 atomes de carbone, ou forment ensemble un noyau carbocyclique pentagonal ou hexagonal,
dans une réaction catalysée au palladium dans des solvants inertes,
ou bien
[C3] dans le cas R⁴² = groupes
dans lesquels 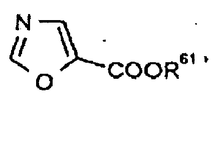
R⁶¹ représente un reste alkyle linéaire ou ramifié ayant jusqu'à 4 atomes de carbone,
des composés de formule générale (III-VI) 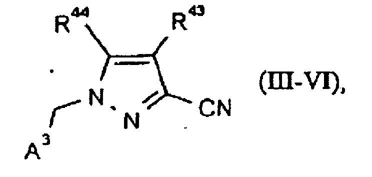 dans laquelle
A³, R⁴³ et R⁴⁴ ont la définition indiquée ci-dessus
sont amenés à réagir avec des composés diazo de formule générale (III-VII) 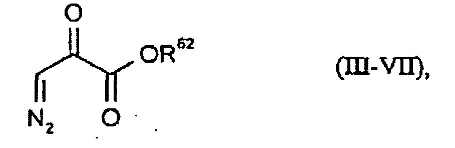 dans laquelle
R⁶² représente un reste alkyle linéaire ou ramifié ayant jusqu'à 4 atomes de carbone,
en présence de sels de cuivre ou de sels de rhodium, pour former des composés de formule générale (III-Ia) 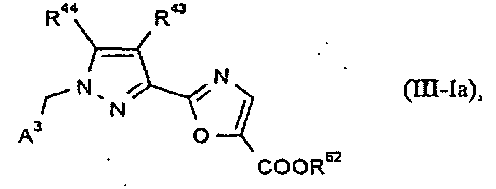 dans laquelle
A³, R⁴³, R⁴⁴ et R⁶² ont la définition indiquée ci-dessus,
[D3] dans le cas R⁴² = 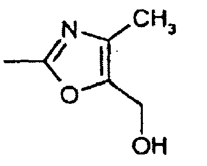 des composés de formule générale (III-VIII) 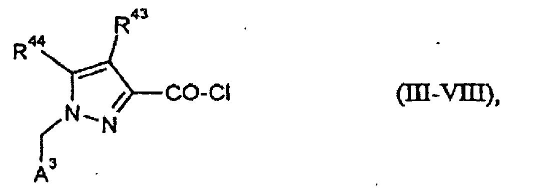 dans laquelle
A³, R⁴³ et R⁴⁴ ont la définition indiquée ci-dessus,
sont transformés directement par réaction avec le composé de formule (III-IX) 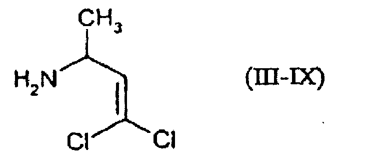 dans le système NaOCO-CH₃/N-méthylpyrrolidine en les composés de formule générale (III-Ib) 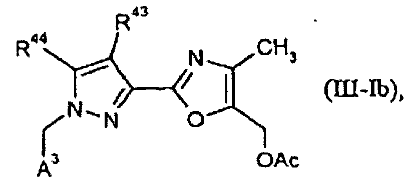 dans laquelle
R^{43'} R⁴⁴ et A³ ont la définition indiquée ci-dessus,
puis le groupe acétyle est éliminé par l'action de l'hydroxyde de potassium dans le méthanol,
ou bien
les composés de formule générale (III-X) 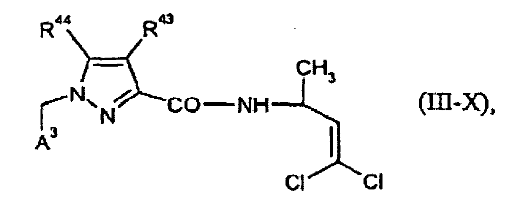 dans laquelle
R⁴³, R⁴⁴ et A³ ont la définition indiquée ci-dessus,
sont tout d'abord préparés par réaction des composés de formule générale (III-VIII) avec le composé de formule (III-IX)
et les composés hydroxyméthylés sont produits dans une autre étape par l'action de l'hydroxyde de potassium,
et transformés le cas échéant, par alkylation selon des méthodes usuelles, en composés alkoxylés correspondants,
ou bien
[E3] des composés de formule générale [III-VII] 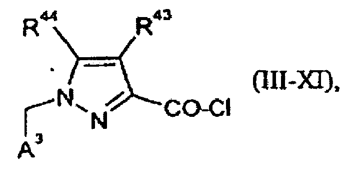 dans laquelle
A³, R⁴³ et R⁴⁴ ont la définition indiquée ci-dessus,
sont transformés par réaction avec le composé de formule (III-XII) 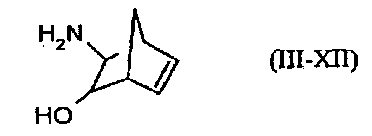 en composés de formule générale (III-XIII) 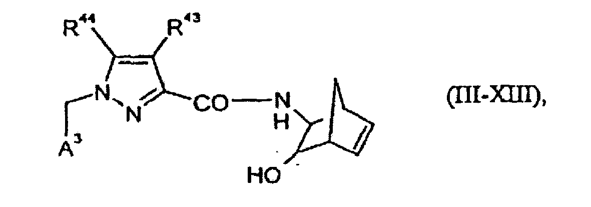 dans laquelle
A³, R⁴³ et R⁴⁴ ont la définition indiquée ci-dessus,
puis amenés à réagir dans le sens d'une rétro-réaction de Diels-Alder
ou bien
[F3] des composés de formule générale (III-XIV) 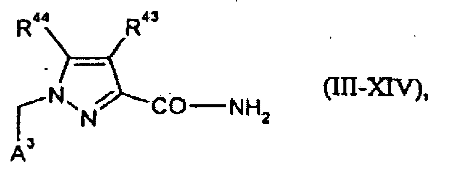 dans laquelle
A³, R⁴³ et R⁴⁴ ont la définition indiquée ci-dessus, sont amenés à réagir avec des composés de formule générale (III-XV)
Br-CH₂-CO-R⁶³ (III-XV)
dans laquelle
R⁶³ représente un reste alkyle ou un reste alkoxycarbonyle linéaire ou ramifié ayant chacun jusqu'à 4 atomes de carbone,
dans des solvants organiques pour former des composés de formule générale (III-Ic) 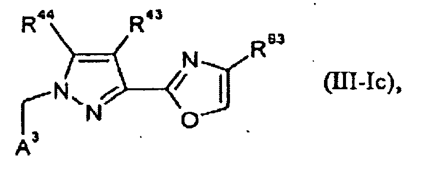 dans laquelle
A³, R⁴³, R⁴⁴ et R⁶³ ont la définition indiquée ci-dessus,
et dans cas de l'ester (R⁶³ = CO₂-(alkyle en C₁ à C₄), une réduction en composés hydroxyméthylés correspondants est effectuée selon des méthodes usuelles,
ou bien
[G3] dans le cas R⁴² = 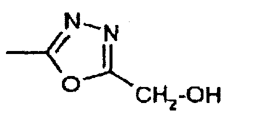 des acides carboxyliques de formule générale (III-XVI) 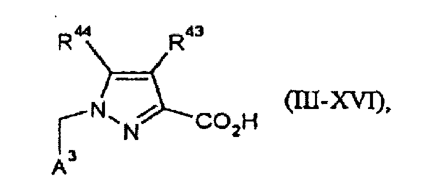 dans laquelle
A³, R⁴³ et R⁴⁴ ont la définition indiquée ci-dessus,
sont tout d'abord transformés avec l'hydrate d'hydrazine en composés de formule générale (III-XVII) 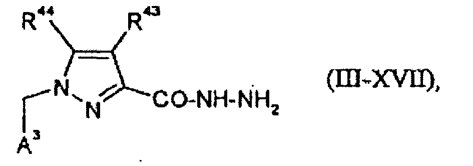 dans laquelle
A³, R⁴³ et R⁴⁴ ont la définition indiquée ci-dessus, dans une autre étape avec le composé de formule (III-XVIII)
Cl-CO-CH₂-Cl (III-XVIII)
les composés de formule générale (III-XiX) 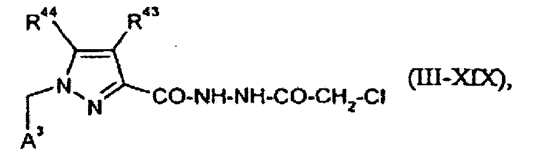 dans laquelle
A³, R⁴³ et R⁴⁴ ont la définition indiquée ci-dessus, sont produits
puis une cyclisation en composés de formule générale (III-Id) 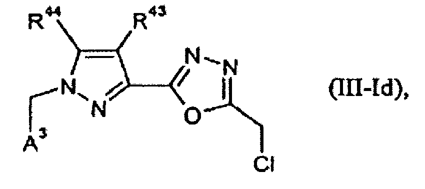 dans laquelle
A³, R⁴³ et R⁴⁴ ont la définition indiquée ci-dessus,
est conduite sous l'action de l'oxytrichlorure de phosphore et les composés à substituant -CH₂-OH sont produits comme déjà décrit ci-dessus avec passage par le stade des composés à substituant -CH₂-O-CO-CH₃ correspondants,
ou bien
[H3] au cas où R⁴² représente un reste de formule 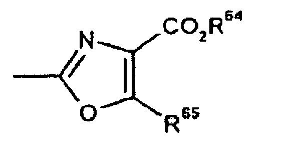 dans laquelle
R⁶⁴ représente l'hydrogène ou un reste alkyle linéaire ou ramifié ayant jusqu'à 4 atomes de carbone et
R⁶⁵ comprend les limites de définition des sous-substituants indiqués pour le reste hétérocyclique R⁴², des composés de formule générale (III-XX) 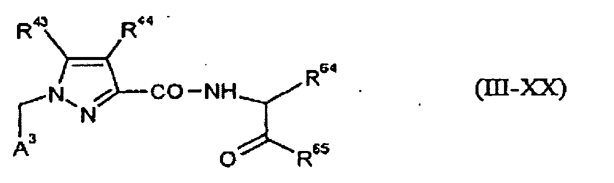 dans laquelle
A³, R⁴³, R⁴⁴, R⁶⁴ et R⁶⁵ ont la définition indiquée ci-dessus, sont amenés à réagir dans le système PPh₃/I₂ en présence d'une base, avantageusement avec la triéthylamine
ou bien
[I3] au cas où R⁴² représente le groupe de formule 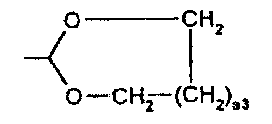 dans laquelle a³ a la définition indiquée ci-dessus,
des composés de formule générale (III-XXI) 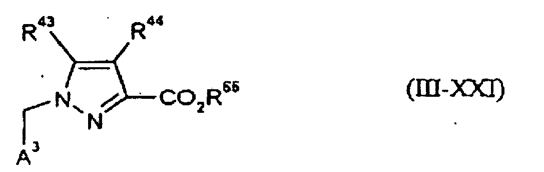 dans laquelle
A³, R⁴³ et R⁴⁴ ont la définition indiquée ci-dessus et
R⁶⁶ a la définition indiquée ci-dessus pour R⁶⁴ et y est identique ou en est différent
sont tout d'abord transformés par réduction selon des méthodes usuelles en composés de formule générale (III-XII) 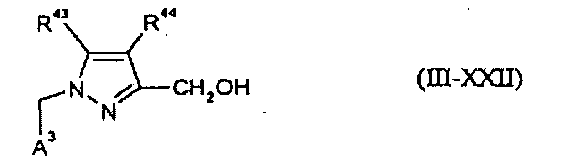 dans laquelle
A³, R⁴³ et R⁴⁴ ont la définition indiquée ci-dessus,
puis les composés de formule (III-XXIII) 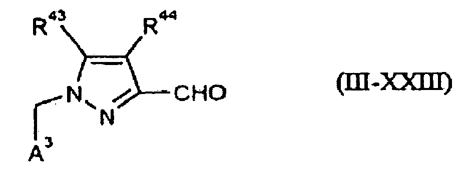 dans laquelle
A³, R⁴³ et R⁴⁴ ont la définition indiquée ci-dessus, sont produits par oxydation, ou bien
les composés de formule générale (III-XXI) sont transformés directement en composés de formule générale (III-XXIII) par réduction
et finalement amenés à réagir selon des méthodes classiques avec des composés 1,2- ou 1,3-dihydroxylés
ou bien
[J3] au cas où R⁴² représente le reste de formule 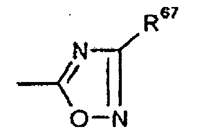 dans laquelle
R⁶⁷ a la définition indiquée ci-dessus pour R⁶⁵ et y est identique ou en est différent,
des composés de formule générale (III-XXIV) 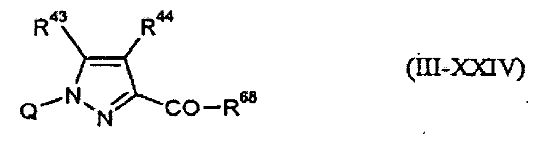 dans laquelle
R⁴³ et R⁴⁴ ont la définition indiquée ci-dessus,
Q représente l'hydrogène ou le reste -CH₂-A³ et
R⁶⁸ représente un halogène ou un reste alkoxy linéaire ou ramifié ayant jusqu'à 4 atomes de carbone, avantageusement le chlore, un reste méthoxy ou éthoxy,
sont amenés à réagir avec des composés de formule générale (III-XXV) 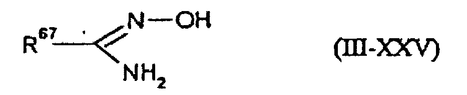 dans laquelle
R⁶⁷ a la définition indiquée ci-dessus
le cas échéant en présence d'une base puis, au cas où Q représente H, amenés à réagir avec des composés de formule générale A³-CH₂-Br (III-XXVI), dans laquelle A a la définition indiquée ci-dessus, ou bien
des composés de formule générale (III-XXVII) 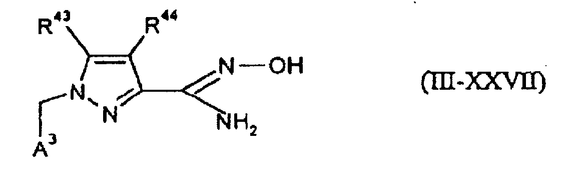 dans laquelle
A³, R⁴³ et R⁴⁴ ont la définition indiquée ci-dessus, sont amenés à réagir avec des composés de formule générale (III-XXVIII)
R^{67'}-CO-R^{68'} (III-XXVII)
dans laquelle
R^{67'} a la définition indiquée ci-dessus pour R⁶⁷ et y est identique ou en est différent
et
R^{68'} a la définition indiquée ci-dessus pour R⁶⁸ et y est identique ou en est différent,
éventuellement en présence d'une base,
et dans le cas des restes -S(O)_{c3}NR⁵⁰R⁵¹ et -S(O)_{c3}NR^{50'}R^{51'}, on conduit à partir des composés non substitués de formule générale (III-I) tout d'abord une réaction avec le chlorure de thionyle et finalement avec le composant amine,
et le cas échéant, on fait varier ou on introduit les substituants indiqués pour R⁴², R⁴³, R⁴⁴ et/ou A³ selon des méthodes usuelles, avantageusement par réduction, oxydation, élimination de groupes protecteurs et/ou substitution nucléophile.

3. Médicament contenant au moins un composé de formule générale (III-I) suivant la revendication 1.

4. Préparations médicamenteuses contenant une combinaison d'au moins un composé de formule générale (III-I) suivant la revendication 1 et d'au moins un nitrate organique ou un donneur de NO.

5. Préparations médicamenteuses contenant une combinaison d'au moins un composé de formule générale (III-I) suivant la revendication 1 et de composés qui inhibent la dégradation du monophosphate de guanosine cyclique (GMPc).

6. Utilisation de composés de formule générale (III-I) suivant la revendication 1 dans la préparation de médicaments destinés au traitement de maladies cardio-vasculaires.

7. Utilisation de composés de formule générale (III-I) suivant la revendication 1 dans la préparation de médicaments destinés à la prophylaxie et au traitement des séquelles de survenues d'infarctus cérébraux (apoplexia cerebri) telles qu'aploxie cérébrale, ischémies cérébrales et traumatismes cranio-cérébraux.
